# EUROPEAN PATENT APPLICATION

(11) **EP 4 682 170 A1**
(43) Date of publication of application: **21.01.2026**
(21) Application number: 24771167.4
(22) Date of filing: 12.03.2024
(51) Int. Cl.: C07K 16/28, A61P 35/00, A61K 39/00

(54) **NOVEL CD3-BINDING ANTIBODY AND MULTISPECIFIC ANTIBODY COMPRISING SAME**

(30) Priority: 13.03.2023 KR 20230032716
(71) Applicant: Mustbio Co., Ltd., Seoul 08390 (KR)
(72) Inventor: JUNG, Sung Youb, Suwon-si, Gyeonggi-do 16507 (KR); BANG, Hyo Joo, Ansan-si, Gyeonggi-do 15578 (KR); PARK, Young Jin, Suwon-si, Gyeonggi-do 16509 (KR); JUNG, Yong Jun, Gyeonggi-do 06990 (KR); KIM, Mi Seong, Suwon-si, Gyeonggi-do 16509 (KR); KIM, Sun A, Gunpo-si, Gyeonggi-do 15863 (KR); KIM, Su Yoon, Seoul 08538 (KR)
(74) Representative: EIP
(86) International application number: PCT/KR2024/003159
(87) International publication number: WO 2024/191168

(57) **Abstract**

The present disclosure relates to a novel human CD3-binding antibody with increased safety and a multispecific antibody using the same. The multispecific antibody activates the immune system, but compared to existing CD3-binding antibodies, significantly lowers the release of cytokines, while maintaining cell-killing ability and having a high level of stability, and thus it can be used in diagnosis and treatment of various diseases, including cancer.

## Description

### Technical Field

The present application claims priority to Korean Patent Application No. 10-2023-0032716, filed on March 13, 2023, the disclosure of which is incorporated by reference in its entirety.

The present disclosure relates to a novel CD3-binding antibody and a multispecific antibody including the same.

### Background Art

When an antigen originating from outside the body enters the body, a series of processes occur in the immune system, initiated by recognition of antigens by antigen-presenting cells (APCs) and binding of these antigens to major histocompatibility complex (MHC) molecules, which are then delivered in the form of small peptides to T cells. T cells playing a central role in immune responses have T cell receptors specific to various antigens, and only T cells specific to antigen-derived peptides delivered by APCs are activated. Subsequently, secretion of cytokines necessary for immune responses and proliferation of clones of activated T cells occur. In particular, when T cell receptors (TCRs) expressed on the surface of T cells recognize the antigens bound to the MHC molecules, TCRs trigger the secretion of cell-killing factors for APCs and pro-inflammatory factors, to protect the body.

The activation of T cells promotes multiple signaling cascades that ultimately determine cell fate by producing cytokines and regulating survival, proliferation, and differentiation of cells. Early events of the activation of TCRs include phosphorylation of immunoreceptor tyrosine-based activation motifs (ITAMs) located in the cytoplasmic region of a TCR/CD3 complex by a lymphocyte-specific protein tyrosine kinase (Lck). CD3 is, in relation to a T cell receptor complex, a heterodimeric antigen expressed in T cells, and is necessary for the activation of T cells. CD3 is formed by dimeric binding of two among four different chains (CD3ε, CD3γ, CD3δ, and CD3ζ). Such CD3 dimer arrangement includes gamma/epsilon and delta/epsilon. Phosphorylated ITAM triggers binding of zeta-chain-associated protein kinase 70 (ZAP-70) to the TCR/CD3 complex, promoting recruitment and phosphorylation of downstream adapters or scaffold proteins. The Linker for activation of T cells (LAT) and the leukocyte protein SLP-76 (e.g., SH2-domain-containing leukocyte protein of 76 kDa) are the two key substrates of ZAP-70. Phosphorylated LAT and SLP-76 lead to recruitment of many different proteins associated with calcium mobilization, the Ras signaling pathway, and cytoskeletal reorganization. Such signaling cascades induce calcium mobilization, Ras activation, and cytoskeletal reorganization, which are necessary for the growth and differentiation of cells and the secretion of cell-killing substances, as well as activation of specific transcription activators.

As such, antigen-derived activation of T cells is known to exhibit a strong antitumor effect under various experimental conditions. In particular, independent activation of CD3 receptors only has been proposed in terms of a therapeutic target for various diseases because T cells can be activated only through activation of the CD3 receptors without a process of recognizing specific antigens. In particular, a multispecific antibody that simultaneously includes a CD3-binding antibody and a site binding to a tumor-association antigen (TAA) that is highly expressed in cancer cells activates T cells regardless of the TCR specificity of immune cells, causing specific lysis of cancer cells carrying individual TAA, thereby exhibiting effective anticancer activity.

Removab (Catumaxomab, Trion Pharma), the first approved CD3-binding antibody-based multivalent antibody, was approved as a treatment for malignant ascites in Europe in 2009 based on the mechanism of binding simultaneously to the epithelial cell adhesion molecule (EpCAM), which is a cancer cell-specific antigen, and CD3 on immune cells, thereby directing immune cells to target cancer cells and inducing death of cancer cells. However, Removab has a rodent sequence, and thus exhibits immunogenicity when administered to humans. Due to its strong binding strength to CD3 (4.4 nM), it causes a cytokine release syndrome (CRS), which is characterized by excessive secretion of cytokines due to hyperactivation of T cells, and thus, Removab was withdrawn from the market (Ma et al (2021) Frontiers in immunology 12, article 626616).

The first commercially successful CD3-binding antibody-based multivalent antibody is Amgen's Blincyto (Blinatumomab). In 2014, Blincyto was approved as a treatment for a patient with non-Hodgkin's lymphoma by targeting CD3 and CD19 antigens present in B cells at the same time, and enabling T cells of the patient to recognize malignant B cells and exhibit a cytotoxic effect on leukemia cells. Blincyto consists of a CD3-binding antibody single-chain variable fragment (scFv) and a CD19-binding antibody scFv, and the absence of an Fc fusion protein results in an inconvenient need for multiple intravenous (IV) infusions due to short half-life. The CRS side effects caused by hyperactivation of T cells have still emerged in clinical trials (Drawdy et al (2019) J Hematol Oncol Pharma 9(2):38-46). Accordingly, only doses less than those required for pharmacological action could be used, resulting in a decrease in efficacy.

Despite the high potential of CD3-binding antibodies as therapeutic agents through increased immune activation as described above, it is difficult to achieve optimal efficacy due to toxicity and side effects. To overcome this difficulty, it is highly necessary to develop a new treatment method that maintains the cell-killing ability through effective activation of T cells while minimizing side effects by reducing release levels of cytokines as compared to existing CD3-binding antibodies, by using a novel CD3-binding antibody. Such a newly invented CD3-binding antibody may form a multispecific antibody with an antibody binding to various tumor-specific antigens (TSAs) or tumor-association antigens (TAAs), an immune checkpoint inhibitor, a cytokine, an antagonist of a growth factor inhibiting anticancer activity, such as vascular endothelial growth factor (VEGF) and transforming growth factor beta (TGF-β), and immune cell receptor-binding proteins such as CD28 and 41BB, enabling its use in the treatment of individual cancer types with diverse antigens.

### Disclosure of Invention

### Technical Problem

One aspect is to provide a novel CD3 antibody or an antigen-binding fragment thereof that lowers release of cytokines while maintaining cancer cell-killing activity and thus having increased safety, compared to existing CD3 antibodies (also referred to as CD3-binding antibodies).

Another aspect is to provide a protein complex including a first polypeptide including a first CH3 antibody constant region and a second polypeptide including a second CH3 antibody constant region, wherein the first polypeptide and the second polypeptide form a heterodimer.

Another aspect is to provide a multispecific antibody including the CD3 antibody or an antigen-binding fragment thereof.

Another aspect is to provide a method of producing a protein or protein complex, the method including transfecting a cell with an expression vector that encodes the protein or protein complex.

Another aspect is to provide a pharmaceutical composition for preventing or treating a cancer, the pharmaceutical composition including the multispecific antibody as an active ingredient.

### Solution to Problem

One aspect may provide a CD3 antibody or an antigen-binding fragment thereof, including a heavy chain variable region and a light chain variable region that are represented by the following formulas. The heavy chain variable region is indicated by Formula 1, and the light chain variable region is indicated by Formula 2:
- Formula 1 (heavy chain variable region): 
EVQLVESGGGLVQPGGSLRLSCAASGX1SFTGYTMNWVRQAPGKX2LEWVALI
NPYKGVTTYADSVKGRFTISX3DX4SKNTX5YLQMNSLRAEDTAVYYCARSGYYG
DSDWYFDVWGQGTLVTVSS;
 and
- Formula 2 (light chain variable region): 
DIQMTQSPSSLSASVGDRVTITCRASQDIRNYLNWYQQKPGKAPKLLIYYTSX6L
ESGVPSRFSGSGSGTDYTLTISSLQPEDFATYYCQQGNTLPWTFGX7GTKVEIK
RT,
   wherein X₁ to X₅ in the heavy chain variable region may each be selected from the group consisting of tyrosine (Y), glycine (G), cysteine (C), alanine (A), aspartic acid (D), glutamine (Q), valine (V), lysine (K), arginine (R), phenylalanine (F), asparagine (N), serine (S), and leucine (L), and
   wherein X₆ and X₇ in the light chain variable region may each be selected from the group consisting of tyrosine (Y), glycine (G), cysteine (C), alanine (A), aspartic acid (D), glutamine (Q), valine (V), lysine (K), arginine (R), phenylalanine (F), asparagine (N), serine (S), and leucine (L).

In the present specification, the positions of the amino acids are based on EU index of Kabat.

In an embodiment, X₁ to X₅ in the heavy chain variable region may each be selected from the group consisting of tyrosine (Y), phenylalanine (F), glycine (G), valine (V), arginine (R), lysine (K), asparagine (N), alanine (A), aspartic acid (D), glutamine (Q), leucine (L), and cysteine (C) and X₆ and X₇ in the light chain variable region may each be selected from the group consisting of arginine (R), serine (S), glutamine (Q), and cysteine (C).

In another embodiment, in the heavy chain variable region, X₁ may be phenylalanine (F) or tyrosine (Y); X₂ may be glycine (G) or cysteine (C); X₃ may be valine (V), arginine (R), alanine (A), aspartic acid (D), or glutamine (Q); X₄ may be lysine (K) or asparagine (N); and X₅ may be alanine (A) or leucine (L), and in the light chain variable region, X₆ may be arginine (R) or serine (S); and X₇ may be glutamine (Q) or cysteine (C).

In another embodiment, in the heavy chain variable region, X₁ may be phenylalanine (F); X₂ may be glycine (G); X₃ may be arginine (R); X₄ may be asparagine (N); and X₅ may be leucine (L), and in the light chain variable region, X₆ may be serine (S); and X₇ may be glutamine (Q).

In another embodiment, in the heavy chain variable region, X₁ may be phenylalanine (F); X₂ may be cysteine (C); X₃ may be arginine (R); X₄ may be asparagine (N); and X₅ may be leucine (L), and in the light chain variable region, X₆ may be serine (S); and X₇ may be cysteine (C).

In another embodiment, in the heavy chain variable region, X₁ may be phenylalanine (F); X₂ may be glycine (G); X₃ may be alanine (A); X₄ may be asparagine (N); and X₅ may be leucine (L), and in the light chain variable region, X₆ may be serine (S); and X₇ may be glutamine (Q).

In another embodiment, in the heavy chain variable region, X₁ may be phenylalanine (F); X₂ may be cysteine (C); X₃ may be alanine (A); X₄ may be asparagine (N); and X₅ may be leucine (L), and in the light chain variable region, X₆ may be serine (S); and X₇ may be cysteine (C).

In another embodiment, the antibody may include at least one polypeptide selected from the group consisting of polypeptides represented by SEQ ID NOs: 1 to 10. Specifically, the heavy chain variable region of the antibody may include at least one selected from the group consisting of CD3 antibody heavy chain variable region SEQ ID NOs: 1 to 7 as shown in Table 1. In addition, the light chain variable region of the antibody may include at least one selected from CD3 antibody light chain variable region SEQ ID NOs: 8 to 10 shown in Table 1. Although not limited thereto, the polypeptide included in the CD3 antibody may have at least 80 %, 85 %, 90 %, 95 %, 96 %, 97 %, 98 %, or 99 % homology with the SEQ ID NOs: 1 to 10. The polynucleotide sequences encoding the polypeptide are as shown in Table 1.

In addition, the antibody may be a CD3 antibody consisting of the heavy chain variable region and the light chain variable region of SEQ ID NO: 1 and SEQ ID NO: 8, respectively, and may be a CD3 antibody consisting of the heavy chain variable region and the light chain variable region of: SEQ ID NO: 2 and SEQ ID NO: 9, respectively; SEQ ID NO: 3 and SEQ ID NO: 10, respectively; SEQ ID NO: 4 and SEQ ID NO: 9, respectively; SEQ ID NO: 5 and SEQ ID NO: 10, respectively; SEQ ID NO: 6 and SEQ ID NO: 9, respectively; and SEQ ID NO: 7 and SEQ ID NO: 9, respectively.

**[Table 1]**

| No. of CD3-binding antibody variable region | SEQ ID NO. of heavy chain variable region (SEQ ID NO. of polynucleotide encoding the same) | SEQ ID NO. of light chain variable region (SEQ ID NO. of polynucleotide encoding the same) |
|---|---|---|
| CD3-binding antibody variable region 1 | 1(22) | 8(29) |
| CD3-binding antibody variable region 2 | 2(23) | 9(30) |
| CD3-binding antibody variable region 3 | 3(24) | 10(31) |
| CD3-binding antibody variable region 4 | 4(25) | 9(30) |
| CD3-binding antibody variable region 5 | 5(26) | 10(31) |
| CD3-binding antibody variable region 6 | 6(27) | 9(30) |
| CD3-binding antibody variable region 7 | 7(28) | 9(30) |

The CD3 antibody may be a CD3 antibody with similar cell-killing ability but lower cytokine release ability and higher safety, compared to commercially available monoclonal antibodies or bispecific antibodies based on UCHT1 and OKT3 clones. The CD3 antibody may activate T cells without specific antigen recognition by binding to CD3 on a TCR/CD3 receptor on the surface of T cells. CD3-binding antibodies may activate T cells with different functional outcomes depending on their binding affinity. CD3-binding antibodies having high affinity, such as OKT3 and UCHT1 clones, demonstrate high activation of T cells and subsequent death of cancer cells, along with a high level of release of pro-inflammatory cytokines such as interferon gamma (IFNγ), when co-cultured with peripheral blood mononuclear cells (PBMCs) including human T cells and cancer cells. Such high release of cytokines may lead to cytokine release syndrome (CRS), which may present with symptoms such as fever, joint pain, vomiting, diarrhea, and tachycardia, and in severe cases, shock, seizures, delirium, impaired consciousness, and death. The CD3 antibody described in the present specification exhibits low CD3 affinity, yet when compared to monoclonal antibodies or bispecific antibodies based on UCHT1 and OKT3 clones in co-culture with PBMCs and cancer cells, it was found to induce similar cancer cell-killing activity while causing low interferon gamma release. Therefore, the CD3 antibody may be a novel antibody with improved safety compared to existing antibodies.

The sequence of the CD3 antibody described in the present disclosure may be derived from a phage carrying a human antibody library, or may be derived from a rodent into which a human antibody library has been transformed, or may be derived from amino acid substitutions by rational design of the CDR sequence and FR sequence of an existing CD3-binding antibody.

The sequence of the CD3 antibody described in the present disclosure may be converted into various forms of antibodies or antibody fragments thereof, such as Fab, single domain, scFv, and the like. The single domain antibody is not a typical antibody consisting of a heavy chain and a light chain, but rather an antibody consisting solely of a heavy chain or a light chain - for example, antibodies produced by camelids such as camels and llamas consist solely of two heavy chains.

The single chain variable fragment (single chain Fv, scFv) is a fusion of variable regions of the heavy chain and light chain, in which 5 to 25 amino acids are linked together via a short peptide linker, wherein the linker may typically include not only glycine for flexibility but also serine or threonine for solubility.

In the present specification, the term "antibody" may be used interchangeable with the term "immunoglobulin (Ig)". A complete antibody has a structure including two full-length light chains and two full-length heavy chains, wherein each light chain is linked to the heavy chains via disulfide bonds (SS-bonds). There are two types of light chains, λ and κ, consisting of approximately 211 to 217 amino acids. In each human antibody, only one type of light chains exists in the same manner. The light chain consists of a succession of a constant region and a variable region. There are five types (γ, δ, α, µ, and ε) of heavy chain chains, and the heavy chain determines the type of antibodies. Types α and γ consist of 450 amino acids, and types µ and ε consist of 550 amino acids. The heavy chain have two regions, i.e., a variable region and a constant region. The variable region refers to a region in an antibody where an antigen binds. The variable region may include a complementarity determining region (CDR) that confers antigen-binding specificity.

The antibody may be, for example, IgA, IgD, IgE, IgG, or IgM. The antibody may be a monoclonal antibody or a polyclonal antibody. The antibody may be an animal-derived antibody, a mouse-human chimeric antibody, a humanized antibody, or a human antibody.

In the present specification, the term "antigen-binding fragment" is a fragment of the entire structure of an immunoglobulin, and refers to a portion of a polypeptide including a site where an antigen can bind. For example, although not limited thereto, the antigen-binding fragment may be scFv, (scFv)2, Fv, Fab, Fab', Fv F(ab')2, or a combination thereof.

The antibody may include an antigen-binding fragment (Fab) region and a fragment crystallizable (Fc) region that binds to a cell surface receptor. When cleaved with papain, a complete antibody may be cleaved into two Fab regions and one Fc region. The Fab region may be a region in which a polypeptide including a variable region domain of the heavy chain (VH) and a first constant region domain of the heavy chain (CH1) and a polypeptide including a variable region domain of the light chain (VL) and a constant region of the light chain (CL) are linked by a SS-bond. The Fc region may be a region in which two polypeptides are linked together, each including a second constant region domain of the heavy chain (CH2) and a third constant region domain of the heavy chain (CH3). The Fc region may form a hinge region.

In addition, the CD3 antibody may be a dual-chain antibody or a single-chain antibody, and the single-chain CD3 antibody may include a domain in which the heavy chain variable region and the light chain variable region are linked together via a linker or carrier. The linker may include a polypeptide represented by SEQ ID NO: 13 or 14.

The CH3 antibody constant region may refer to a third constant region domain of the heavy chain constant domain of the antibody.

The CH3 antibody constant region may be modified to reduce binding strength to Fcγ receptors or to have high stability by eliminating or significantly reducing effector functions. In addition, the CH3 antibody constant region may be modified to reduce antibody-dependent cellular cytotoxicity (ADCC).

The first polypeptide and the second polypeptide may form the Fc region of the antibody.

The structure of the antibody described in the present specification consists of variable regions and constant regions of the heavy chain and light chain, and the variable region may further consist of a complementarity determining region (CDR) that directly binds to an antigen and a frame region (FR) that supports a CDR loop. The antibody described in the present specification provides a CDR sequence and an FR sequence of a novel CD3-binding protein.

Typically, the effector functions of the antibody are mediated by the Fc region, which binds to its receptor, Fcγ receptor or complement component molecule, C1q, thereby inducing ADCC or complement-dependent cytotoxicity (CDC). Immune cells have many Fcγ receptors such that unpreferable death of immune cells may be induced. Thus, it is preferable to eliminate the effector functions. Therefore, the CH3 antibody constant region may be modified to reduce binding strength to Fcγ receptors or to have high stability by eliminating or significantly reducing the effector functions. In addition, the CH3 antibody constant region may be modified to reduce ADCC.

Another aspect is to provide a protein complex including a first polypeptide having a first CH3 antibody constant region and a second polypeptide having a second CH3 antibody constant region, wherein the first polypeptide and the second polypeptide form a heterodimer.

In an embodiment, at least one selected from the group consisting of an N-terminal and a C-terminal of the first polypeptide may include an immune checkpoint inhibitor, an antibody or an antigen-binding fragment thereof against a tumor-specific antigen or tumor-associated antigen, a cytokine, a hormone, or an immune cell receptor, and
at least one selected from the group consisting of an N-terminal and a C-terminal of the second polypeptide may include the CD3 antibody of claim 1, an immune checkpoint inhibitor, an antibody or an antigen-binding fragment against a tumor-specific antigen or tumor-associated antigen, a cytokine, a hormone or an immune cell receptor.

The immune checkpoint inhibitor may be, for example, PD-L1, PD-1, LAG3, VISTA, BTLA, TIM3, TIGIT, CTLA-4, A2aR (adenosine A2a receptor), 2B4 (CD244), B- and T-lymphocyte attenuator (BTLA, CD272), and the like, but is not limited thereto.

In another embodiment, the CH3 antibody constant region may include at least one amino acid selected from the group consisting of alanine (A), glycine (G), glutamine (Q), phenylalanine (F), glutamic acid (E), and serine (S) at least one position selected from positions 234, 235, 329, 297, 331, and 265. For example, the CH3 antibody constant region may include a substitution of leucine (L) at positions 234 and 235 substituted with alanine (A) (L234A/L235A).

In the present specification, '/' may refer to 'and'. For example, the L234A/L235A may refer to a mutant that simultaneously has a mutation in which leucine (L) at position 234 is substituted with alanine (A) and a mutation in which leucine (L) at position 235 is substituted with alanine (A).

In another embodiment, the CH3 antibody constant region may include a substitution of leucine (L) at positions 234 and 235 with alanine (A), and a substitution of proline (P) at position 329 with glycine (G) (L234A/L235A/P329G).

In addition, the CH3 antibody constant region may include a substitution of asparagine (N) at position 297 with alanine (A), glutamine (Q), or glycine (G) (N297A, N297Q, or N297G).

In addition, the CH3 antibody constant region may include a substitution of leucine (L) at positions 234 and 235 with phenylalanine (F) and glutamic acid (E) and a substitution of proline (P) at position 331 with serine (S) (L234F/L235E/P331S).

In addition, the CH3 antibody constant region may include a substitution of leucine (L) at positions 234 and 235 with phenylalanine (F) and glutamic acid (E) and a substitution of aspartic acid (D) at position 265 with alanine (A) (L234F/L235E/D265A).

In another embodiment, the first CH3 antibody constant region may include tryptophan (W) at position 366, and the second CH3 antibody constant region may include serine (S) at position 366, alanine (A) at position 368, and valine (V) at position 407; and the second CH3 antibody constant region may include glycine (G) or phenylalanine (F) at position 351.

In another embodiment, the first CH3 antibody constant region may include serine (S) at position 366, alanine (A) at position 368, and valine (V) at position 407, and the second CH3 antibody constant region may include tryptophan (W) at position 366; and the first CH3 antibody constant region may include glycine (G) or phenylalanine (F) at position 351.

In another embodiment, the first CH3 antibody constant region may include tryptophan (W) at position 366, and the second CH3 antibody constant region may include glycine (G) at position 351, serine (S) at position 366, alanine (A) at position 368, and valine (V) at position 407; and the first CH3 antibody constant region may include phenylalanine (F) or tryptophan (W) at position 351.

In another embodiment, the first CH3 antibody constant region may include glycine (G) at position 351, serine (S) at position 366, alanine (A) at position 368, and valine (V) at position 407, and the second CH3 antibody constant region may include tryptophan (W) at position 366; and the second CH3 antibody constant region may include phenylalanine (F) or tryptophan (W) at position 351.

In another embodiment, the first CH antibody constant region and the second CH antibody constant region may include alanine (A) at positions 234 and 235.

The protein complex may include an element selected from the group consisting of an antigen-binding fragment (Fab), a single chain variable fragment (scFv), an extracellular domain of a membrane receptor, an agonist, an antagonist, a ligand, a decoy receptor, a cytokine, a coagulation factor, and an affinity tag.

In addition, the CD3 antibody may form a bispecific or multispecific antibody with a binding protein, an antibody or an antigen-binding fragment against a tumor-specific antigen (TSA) or a tumor-associated antigen (TAA). The TAA is a protein that is expressed relatively limitedly in tumor cells, and the TSA is a protein that is expressed uniquely in tumor cells.

The TAA is an antigen expressed by the presence of tumors, and may include, for example, EGFR, HGFR, IGF1R, VEGFR, HER-2, B7H3, GPC3, CEA, TROP, or PSMA. The TSA is an antigen expressed only in tumor tissues, and may include AFP or HPV, but is not limited thereto. The TSA may be a protein expressed specifically in target cells, including cancer cells, such as cells in the ovaries, breasts, gastrointestinal track, brain, head and neck, prostate, colon, lungs, kidneys, pancreas, and the like, as well as hematologic tumor, such as B-cell tumor including leukemia, lymphoma, sarcoma, carcinoma, neuron tumor, squamous cell carcinoma, germ cell tumor, metastasis, undifferentiated tumor, seminoma, melanoma, myeloma, neuroblastoma, mixed cell tumor, neoplasia caused by infectious agents, and other malignant tumors, cells infected by pathogens, and autoreactive cells that cause inflammation and/or autoimmunity.

The cytokine may include not only a cytokine itself but also a drug that activates a cytokine receptor, and although not limited thereto, may include at least one or derivative thereof selected from the group of IL2, II15, IL17, IL21, and IL12.

Another aspect is to provide a multispecific antibody including the CD3 antibody or an antigen-binding fragment thereof. The multispecific antibody may include a bispecific antibody.

The multispecific antibody may include: the novel CD3 antibody; a CD antibody binding to various TSAs and TAAs; or an immune checkpoint inhibitor, a cytokine, an antagonist of a growth factor that inhibits anticancer activity, such as vascular endothelial growth factor (VEGF) and transforming growth factor beta (TGF-β), and an immune cell receptor protein complex such as CD28 and 41BB.

When the CD3 antibody forms a multispecific antibody with a different antibody, each Fab or scFv may be bound to the C-terminus or N-terminus of the Fc; each Fab or scFv may be bound via a peptide linker without the Fc; or different Fab or scFv may be tandemly bound to one arm of the Fc, and likewise, the linking form of antigen-binding sites of different antibodies is unlimited.

Another aspect is to provide a method of preparing the multispecific antibody.

Another aspect is to provide a pharmaceutical composition for preventing or treating a cancer, the pharmaceutical composition including the multispecific antibody as an active ingredient.

In the present specification, the term "cancer" refers to a condition in which abnormally hyperproliferation occurs due to a problem in the normal functions of cell division, differentiation, and death, resulting in the formation of a mass that invades surrounding tissues and organs and destroys or alters existing structures.

In addition, although not limited thereto, the cancer may be any one selected from the group consisting of cervical cancer, lung cancer, pancreatic cancer, non-small cell lung cancer, liver cancer, colon cancer, bone cancer, skin cancer, head and neck cancer, cervical cancer, skin melanoma, intraocular melanoma, uterine cancer, ovarian cancer, rectal cancer, brain tumor, bladder cancer, blood cancer, stomach cancer, perianal cancer, breast cancer, fallopian tube carcinoma, endometrial carcinoma, vaginal cancer, vulvar carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, renal cancer, ureteral cancer, renal cell carcinoma, renal pelvis carcinoma, central nervous system (CNS) tumor, primary CNS lymphoma, spinal cord tumor, brain stem glioma, and pituitary adenomas.

In the present specification, the term "prevention" refers to any action that inhibits a disease or delay the onset of a disease by administering a composition. The term "treatment" refers to any action that ameliorates or beneficially changes a symptom of a disease by administering a composition.

The pharmaceutical composition may further include a "pharmaceutically acceptable carrier". The pharmaceutically acceptable carrier that may be generally used in formulation may include saline, sterile water, Ringer's solution, buffered saline, cyclodextrin, a dextrose solution, a maltodextrin solution, glycerol, ethanol, liposome, and the like, but is not limited thereto. As necessary, other conventional additives such as an antioxidant, a buffering solution, and the like may be further included. In addition, a diluent, a dispersant, a surfactant, a binder, and a lubricant may be further added to be formulated into an injectable form, such as an aqueous solution, a suspension, an emulsion, and the like, a pill, a capsule, a granule, or a tablet. A suitable pharmaceutically acceptable carrier and a preparation thereof may be preferably referenced from the method described in the Remington's document according to a component. The pharmaceutical composition of the present disclosure is not particularly limited to a formulation, but may be prepared into injections, inhalations, skin remedies for external use, or oral ingestions.

In the present specification, the term "pharmaceutically acceptable" refers to a composition that is, within the scope of sound medical judgment, suitable for use in contact with the tissues of a subject (e.g., humans) without undue toxicity, irritation, allergic response or other problems or complications, and are commensurate with a reasonable benefit/risk ratio.

The pharmaceutical composition may be administered orally or non-orally (e.g., intravenously, subcutaneously, transdermally, intranasally, or intratracheally) depending on the intended method, and the dosage may vary depending on patient's condition and body weight, disease severity, drug formulation, a route of administration, and timing of administration, but may be appropriately selected by one of ordinary skill in the art.

The pharmaceutical composition may be administered in a pharmaceutically effective amount. In the present disclosure, the "pharmaceutically effective amount" refers to an amount sufficient to treat a disease with a reasonable benefit/risk ratio applicable to medical treatment, and an effective dose level may be determined according to factors, such as a type of a patient's disease, severity, drug activity, drug sensitivity, timing of administration, a route of administration and an excretion rate, a period of treatment, drugs being simultaneously used, and other factors well known in the medical field. The composition according to the present disclosure may be administered as an individual therapeutic agent, administered in combination with other therapeutic agents, administered sequentially or simultaneously with a conventional therapeutic agent, and administered once or multiple times. Considering all of the aforementioned factors, it is important to administer an amount that can achieve maximum effect with a minimum amount without side effects, and such an amount may be easily determined by one of ordinary skill in the art.

An effective amount of the pharmaceutical composition may vary according to a patient's age, gender, and body weight, and generally, the pharmaceutical composition may be administered at 0.001 to 150 mg, preferably, 0.01 to 100 mg, per kg of body weight daily or every two days, or 1 to 3 times daily. However, as the effective amount may be increased or decreased by an administration route, severity of obesity, gender, body weight, or an age, the dose does not limit the scope of the present disclosure in any way.

Another aspect provides a method of preventing or treating a cancer, the method including administering the pharmaceutical composition to a subject. Details of the pharmaceutical composition, administration, prevention and treatment are as described above.

The subject may be mammals, such as humans, cows, horses, pigs, dogs, sheep, goats or cats, or mammals other than humans. The subject may be suffering from cancer or at high risk of developing cancer.

Another aspect provides use of the pharmaceutical composition in the treatment or prevention of cancer. Details of the pharmaceutical composition, prevention and treatment are as described above.

### Advantageous Effects of Invention

The novel CD3-binding antibody according to an aspect may substitute a specific amino acid in a CDR region with a specific amino acid in a frame region, thereby regulating binding to CD8+ T cells. Compared to existing CD3-binding antibodies, the novel CD3-binding antibody maintains the same level of cancer cell-killing activity while keeping the release of cytokines that cause side effects at low levels, thereby increasing anticancer activity. In addition, the pharmaceutical composition including the CD3-binding antibody can reduce side effects and maximize anticancer activity compared to existing antibody therapeutic agents, and may be used for the prevention or treatment of immune diseases of various types of cancer.

### Brief Description of the Drawings

FIG. 1A shows positions of each amino acid in a heavy chain variable region of a CD3-binding antibody. (the positions of all the amino acids are assigned according to the Kabat)
FIG. 1B shows positions of each amino acid in a light chain variable region of a CD3-binding antibody. (the positions of all the amino acids are assigned according to the Kabat.)
FIG. 2A shows results of purification of a protein complex of CD3-binding antibody variable region 3-Fc x PD-L1 antibody, according to an aspect, by using a Protein A affinity column purification method.
FIG. 2B shows results of purification of a protein complex of CD3-binding antibody variable region 3-Fc x PD-L1 antibody, according to an aspect, by using a Phenyl HP hydrophobic interaction column purification method.
FIG. 2C shows results of purity analysis of a protein complex of CD3-binding antibody variable region 3-Fc x PD-L1 antibody, according to an aspect.
FIGS. 3A to 3C show results of identifying binding strengths of a protein complex of CD3-binding antibody variable region 2-Fc x PD-L1 antibody, a protein complex of CD3-binding antibody variable region 3-Fc x PD-L1 antibody, and Avelumab, to a human PD-L1 antigen.
FIGS. 4A to 4C show results of identifying binding strengths of a protein complex of CD3-binding antibody variable region 2-Fc x PD-L1 antibody, a protein complex of CD3-binding antibody variable region 3-Fc x PD-L1 antibody, and a CD3 monoclonal antibody, to human CD3εδ.
FIGS. 5A to 5C show results of identifying binding strengths of a protein complex of CD3-binding antibody variable region 2-Fc x PD-L1 antibody, a protein complex of CD3-binding antibody variable region 3-Fc x PD-L1 antibody, and a CD3 monoclonal antibody, to human CD3εγ.
FIG. 6A shows results of identifying the tumor cell-killing effect of a protein complex of CD3-binding antibody variable region 2-Fc x PD-L1 antibody and a protein complex of CD3-binding antibody variable region 3-Fc x PD-L1 antibody in PD-L1-expressing cancer cells.
FIG. 6B shows results of identifying the levels of cytokine release induced by a protein complex of a CD3-binding antibody variable region 2-Fc x PD-L1 antibody and a protein complex of a CD3-binding antibody variable region 3-Fc x PD-L1 antibody in PD-L1-expressing cancer cells.
FIG. 7 shows results of identifying the release of cytokines induced by a protein complex of CD3-binding antibody variable region 2-Fc x PD-L1 antibody in the presence or absence of cancer cells.
FIGS. 8A to 8C show results of identifying the cell-killing effects of a protein complex of CD3-binding antibody variable region 2-Fc x PD-L1 antibody on cancer cells and normal cells.
FIG. 9 shows results of comparing the anticancer effects of a protein complex of CD3-binding antibody variable region 2-Fc x PD-L1 antibody and positive control groups (Avelumab and a protein complex of CD3 (OKT3)-binding antibody variable region-Fc x PD-L1 antibody) in a humanized breast cancer xenograft mouse model.
FIG. 10 shows results of anticancer activity against colorectal cancer by a protein complex of CD3-binding antibody variable region 2-Fc x PD-L1 antibody in a huCD3 knock-in mouse model.
FIGS. 11A to 11C show results of measuring the level cytokine release induced by a protein complex of CD3-binding antibody variable region 2-Fc x PD-L1 antibody and a protein complex of CD3(OKT3)-binding antibody variable region-Fc x PD-L1 antibody over time in a colorectal cancer-induced animal model using a human CD3 knock-in mouse.
FIG. 12A shows results of purification of a protein complex of CD3-binding antibody variable region 3-Fc x GPC3 antibody by using a Protein A affinity column purification method.
FIG. 12B shows results of purification of a protein complex of CD3-binding antibody variable region 3-Fc x GPC3 antibody by using a Phenyl HP hydrophobic interaction column purification method.
FIG. 12C shows results of purity analysis of a protein complex of CD3-binding antibody variable region 3-Fc x GPC3 antibody, according to an aspect.
FIGS. 13A and 13B show results of identifying binding strengths of a protein complex of a CD3-binding antibody variable region 3-Fc x GPC3 antibody and a CD3 monoclonal antibody to human CD3εδ.
FIGS. 14A to 14C show results of T cell activation of a protein complex of CD3-binding antibody variable region 3-Fc x GPC3 antibody, according to the expression level of GPC3.
FIGS. 15A to 15C show results of identifying the release of off-target cytokines induced by a protein complex of CD3-binding antibody variable region 3-Fc x GPC3 antibody.
FIGS. 16A to 16C show results of identifying the cancer cell-killing effect and release of cytokines induced by a protein complex of CD3-binding antibody variable region 3-Fc x PD-L1 antibody.

### Best Mode for Carrying out the Invention

Hereinafter, preferable examples are presented to help understanding of the present disclosure. However, examples below are only presented only for easier understanding of the present disclosure, and the contents of the present disclosure are not limited by these examples.

### [Examples]

### Example 1. Preparation of protein complex of CD3-binding antibody variable region-Fc x PD-L1 antibody and confirmation of activity of protein complex

### 1-1. Cloning and culturing of protein complex of CD3-binding antibody variable region-Fc x PD-L1 antibody

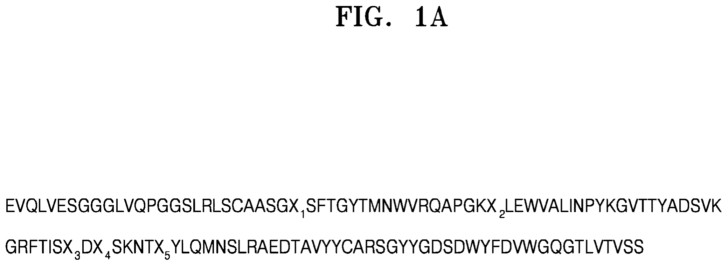

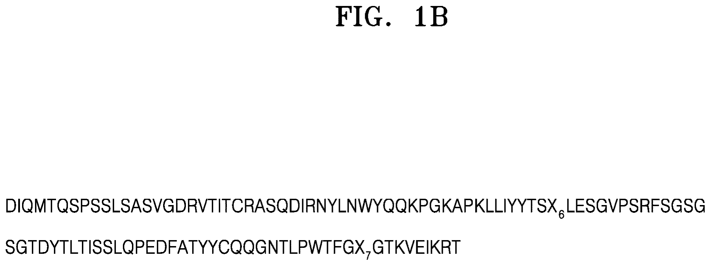

A protein complex including a CD3-binding antibody and a PD-L1 antibody was prepared. A protein complex of CD3-binding antibody variable region-Fc x PD-L1 antibody, which is also referred to as CD3-binding antibody variable region-Fc x PD-L1, refers to a bispecific antibody with a scFv-Fc x Fab-Fc structure in which one arm binds to CD3 and the other arm binds to PD-L1. Specifically, using the Invitrogen GeneArt Gene Synthesis service from ThermoFisher Scientific, the amino acid substitution sites and substituted amino acids in the CD3-binding antibody variable region are each as shown in Table 2. The positions of all the amino acids of the present disclosure are assigned according to the Kabat (Kabat EU index). The position of the heavy chain variable region is shown in FIG. 1A, and the position of the light chain variable region is shown in FIG. 1B.

**[Table 2]**

| | | | Heavy chain variable region | | | | | Light chain variable region | |
|---|---|---|---|---|---|---|---|---|---|
| No. of CD3-binding antibody variable region | Heavy chain variabl e region SEQ ID NO: | Light chain varia ble regio n SEQ ID NO: | X₁ | X₂ | X₃ | X₄ | X₅ | X₆ | X₇ |
| CD3-binding antibody variable region 1 | 1 | 8 | F | | R | N | | | |
| CD3-binding antibody variable region 2 | 2 | 9 | F | | R | N | L | S | |
| CD3-binding antibody variable region 3 | 3 | 10 | F | C | R | N | L | S | C |
| CD3-binding antibody variable region 4 | 4 | 9 | F | | A | N | L | S | |
| CD3-binding antibody variable region 5 | 5 | 10 | F | C | A | N | L | S | C |
| CD3-binding antibody variable region 6 | 6 | 9 | F | | D | N | L | S | |
| CD3-binding antibody variable region 7 | 7 | 9 | F | | Q | N | L | S | |

A polynucleotide encoding a complex including CD3-binding antibody variable regions 1 to 7, CH2 antibody constant region, and CH3 antibody constant region was synthesized, and then inserted into Avrll-BstZ17I enzyme site of pCHO1.0 expression vector to prepare a first expression vector that expresses CD3-binding antibody variable region-Fc. Using the same service, a polynucleotide including Sequence List No. 18 and encoding the heavy chain variable region-heavy chain constant region of the PD-L1 antibody and a polynucleotide including Sequence List No. 19 and encoding the light chain variable region-light chain constant region of the PD-L1 antibody were each synthesized. Next, the polynucleotide encoding the heavy chain variable region-heavy chain constant region of the PD-L1 antibody and the polynucleotide encoding the light chain constant region-light chain variable region of the PD-L1 antibody were inserted to AvrII-BstZ17I enzyme site and EcoRV-Pacl enzyme site of pCHO1.0 expression vector, respectively, to prepare a second expression vector that expresses the PD-L1 antibody. Next, using the ExpiFectamine^{™} CHO Transfection kit (ThermoFisher), the first expression vector and the second expression vector were mixed at a ratio of 1:1 and used for transfecting ExpiCHO-S^{™} cell line. The transfected cells were then cultured for 12 days at 32 °C in a 5 % CO₂ incubator at 120 rpm. 12 days later, the supernatant of the culture medium was separated and collected, and filtered through a sterilized filter to obtain a multispecific antibody including CD3-binding antibody variable region-Fc and anti-PD-L1 Fab-Fc.

### 1-2. Purification of protein complex of CD3-binding antibody variable region-Fc x PD-L1 antibody

The protein complex of CD3-binding antibody variable region-Fc x PD-L1 antibody prepared in Example 1-1 by culturing the cell line was purified to obtain a high-purity substance.

First, the culture medium obtained in Example 1-1 was centrifuged to separate cultured cells from the culture medium. Then, the complex of CD3-binding antibody variable region-Fc x PD-L1 antibody within the separated medium was filtered through a 0.22 µm filter (Thermo Scientific) to remove find debris. The resulting medium from the filtration process was then subjected to first purification using Protein A affinity chromatography (MabSelect PrismA, Cytiva). Specifically, the filterated medium from the filtration process was added to the Protein A column that is stabilized with phosphate buffer saline buffer (pH 7.4), and non-specifically bound proteins were washed off using buffer containing 0.05 M acetic acid and 1 M NaCl as a first wash, and then washed off using 0.2 M acetic acid buffer (pH 5.5) as a second wash. Proteins bound specifically to the Protein A column were eluted using a buffer solution containing 0.02 M acetic acid (pH 3.5), and this sample was neutralized to pH 7.2 using 1 M Tris. To remove precipitates generated during the neutralization process, the neutralized eluate was centrifuged and the supernatant was filtered through a 0.22 µm filter (Thermo Scientific) to remove fine debris.

Subsequently, for second purification intended to remove impurities derived from residual substances in the sample separated from the affinity column, second purification was performed using hydrophobic interaction chromatography (Phenyl **HP,** Cytiva). Specifically, 3.0 M ammonium sulfate (pH 7.0) was added to the sample that has been eluted from the Protein A column and neutralized, and then the resulting solution was titrated with 0.8 M ammonium sulfate (pH 7.0). Next, the resulting sample was added to the phenyl HP column stabilized with buffer (pH 7.0) containing 0.02 M sodium phosphate and 0.8 M ammonium sulfate, and non-specifically bound proteins were washed off using the same buffer. By elution using a buffer solution containing 0.02 M sodium phosphate (pH 7.0) according to an ascending gradient method, the protein complex of CD3-binding antibody variable region-Fc x PD-L1 antibody was obtained. Subsequently, the obtained protein complexes were stabilized with phosphate buffer saline (pH 7.4), and by using size-exclusion high-performance liquid chromatography (HPLC) (TSK-3000SWxL, 7.8 mm x 30 cm, Tosoh), the purity analysis was performed on the purified protein complex of CD3-binding antibody variable region-Fc x PD-L1 antibody.

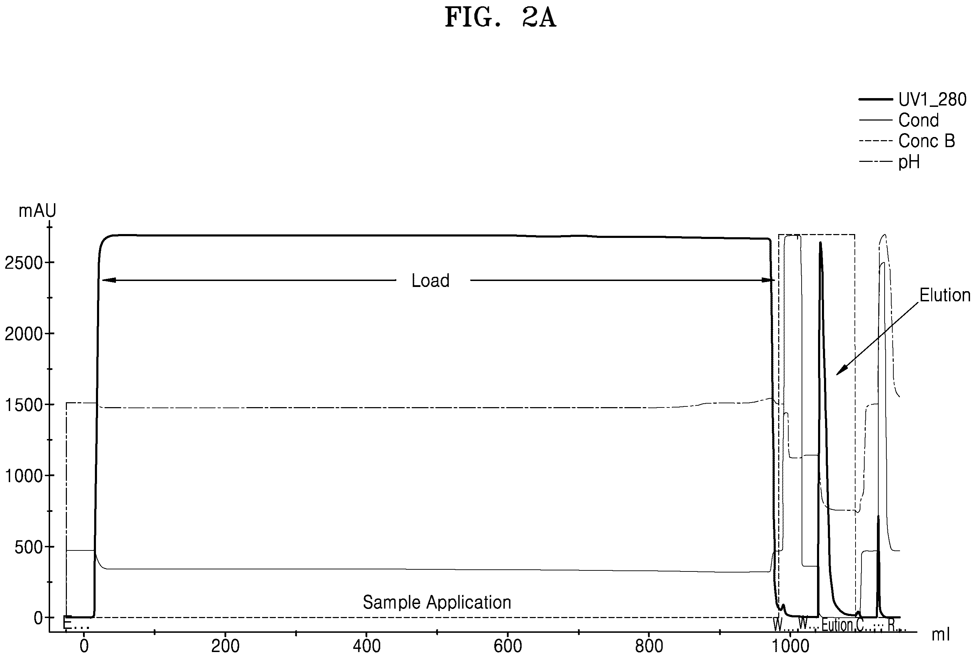

FIG. 2A shows the results of purification of the protein complex of CD3-binding antibody variable region 3-Fc x PD-L1 antibody, according to an aspect, by using the Protein A affinity column purification method.

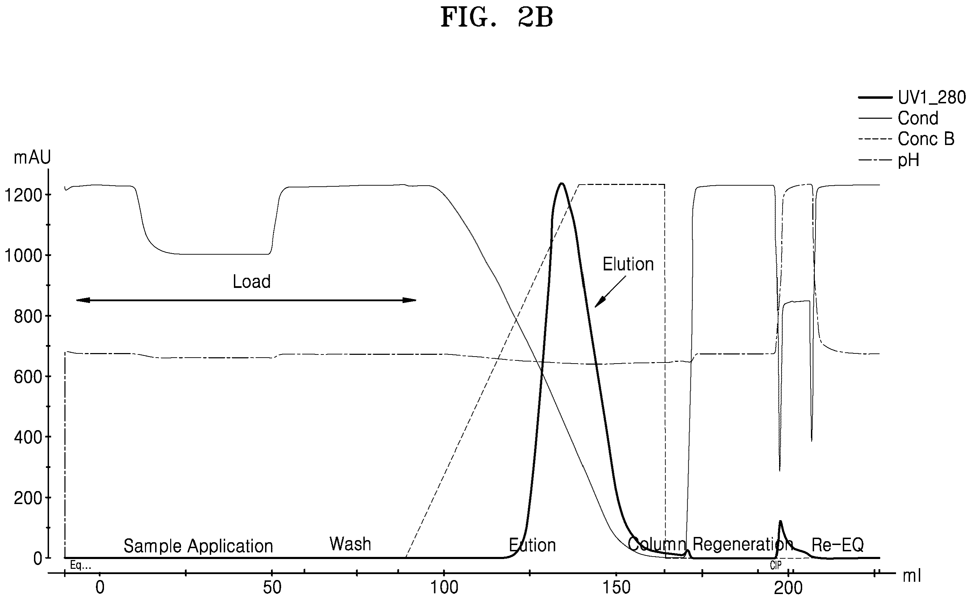

FIG. 2B shows the results of purification of the protein complex of CD3-binding antibody variable region 3-Fc x PD-L1, antibody according to an aspect, by using the phenyl HP hydrophobic interaction column purification method.

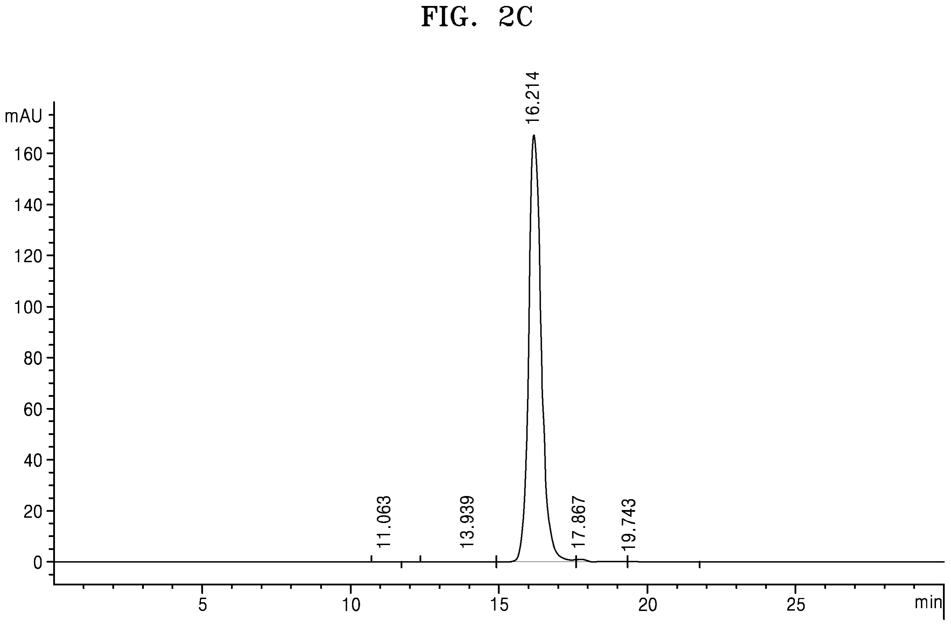

FIG. 2C shows the results of purity analysis of the protein complex of CD3-binding antibody variable region 3-Fc x PD-L1 antibody, according to an aspect.

As a result, as shown in FIG. 2A, eluate peaks of the proteins bound specifically to the column using the elution buffer used in the Protein A affinity column purification method could be observed.

In addition, as shown in FIG. 2B, eluate peaks by the hydrophobic interaction column purification could be observed, which appear after removing impurities remaining in the eluate purified from the Protein A affinity column and non-specifically bound proteins, through the hydrophobic interaction column purification.

In addition, as shown in FIG. 2C, it was confirmed that the purity of the protein complex of CD3-binding antibody variable region 3-Fc x PD-L1 antibody obtained by the Protein A affinity column purification and hydrophobic interaction column purification was at least 98.9 % at a retention time of 16.214 minutes.

### 1-3. Identification of receptor-binding strength of protein complex of CD3-binding antibody variable region-Fc x PD-L1 antibody

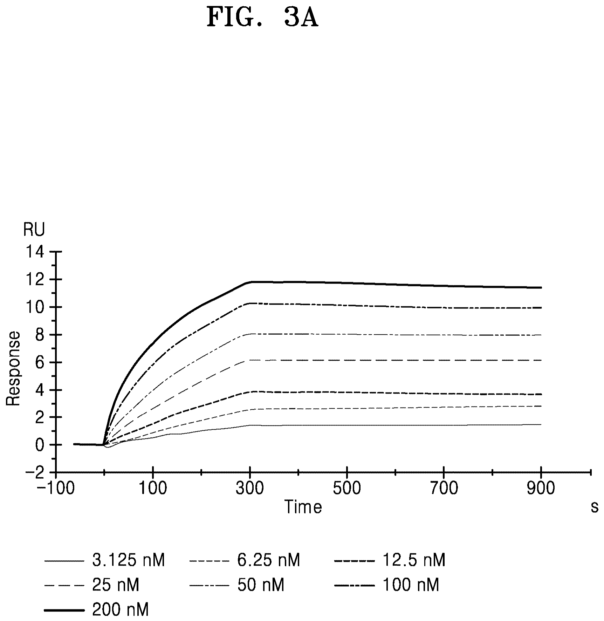

The strengths of the protein complex of CD3-binding antibody variable region 2-Fc x PD-L1 antibody and the protein complex of CD3-binding antibody variable region 3-Fc x PD-L1 antibody to human PD-L1 and a CD3 receptor were confirmed. The binding strength was evaluated by surface plasmon response (SPR) analysis using Biacore T200 (Cytiva, Sweden). Specifically, human PD-L1, human CD3εδ and human CD3εγ were each immobilized on a CM5 sensor chip via covalent bonds. Subsequently, the protein complex of CD3-binding antibody variable region 2-Fc x PD-L1 antibody and the protein complex of CD3-binding antibody variable region 3-Fc x PD-L1 antibody were each applied at various developing concentrations (0.25 to 2000 nM) onto the chip to confirm the binding kinetics for the human PD-L1 and CD3 receptors. Next, the binding affinity (KD) was calculated using the measured association constant (Ka) and dissociation constant (Kd) values. Here, the surface plasmon resonance (SPR) sensorgram analysis was performed using the BIAlogue kinetics evaluation software. For use as positive control groups, Avelumab and a CD3 (UCHT1)-binding monoclonal antibody were used, and the results are shown in Table 3 and 4 and FIGS. 3A to 3C, FIGS. 4A to 4C, and FIGS. 5A to 5C.

**[Table 3]**

| Ligand | Experimental group | Binding affinity (KD, M) |
|---|---|---|
| | Avelumab | 1.44 x 10⁻¹⁰ |
| hPD-L1 | Protein complex of CD3-binding antibody variable region 2-Fc x PD-L1 antibody | 5.64 x 10⁻¹⁰ |
| | Protein complex of CD3-binding antibody variable region 3-Fc x PD-L1 antibody | 3.66 x 10⁻¹⁰ |

FIG. 3A shows the results of identifying binding strength of the protein complex of CD3-binding antibody variable region 2-Fc x PD-L1 antibody to the human PD-L1 antigen.

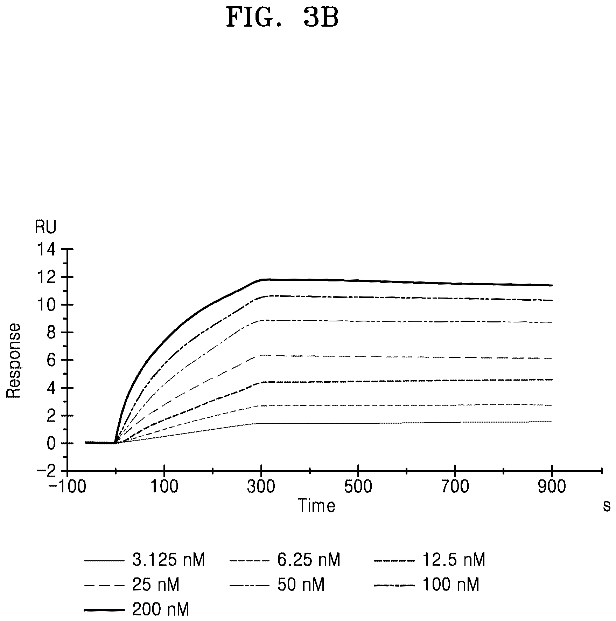

FIG. 3B shows the results of identifying binding strength of the protein complex of CD3-binding antibody variable region 3-Fc x PD-L1 antibody to the human PD-L1 antigen.

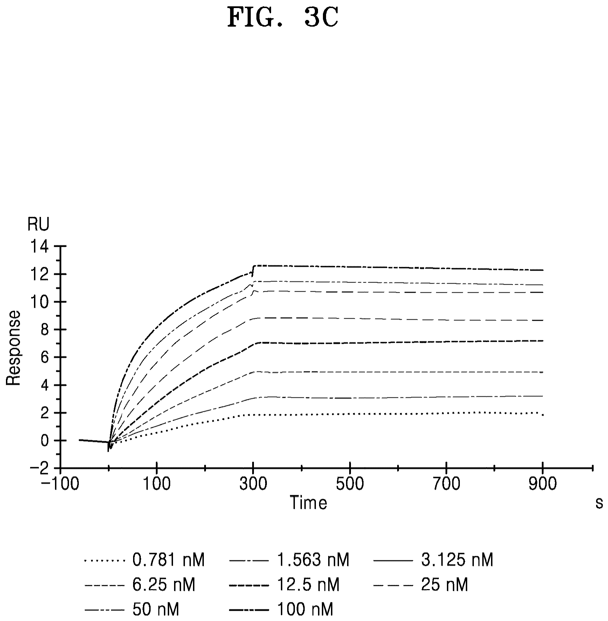

FIG. 3C shows the results of identifying binding strength of Avelumab to the human PD-L1 antigen.

As a result, as shown in FIGS. 3A to 3C and Table 2, it was confirmed that the protein complex of CD3-binding antibody variable region 2-Fc x PD-L1 antibody and the protein complex of CD3-binding antibody variable region 3-Fc x PD-L1 antibody prepared in Example 2 and the positive control group (Avelumab) bind to the human PD-L1 antigen in a concentration-dependent manner. Specifically, it was confirmed that the protein complexes showed the binding strengths of 0.564 nM and 0.366 nM, respectively, showing about 3.9-fold lower binding strength and 2.5-fold lower binding strength, based on the binding strength of the positive group of 0.144 nM.

Referring to the results, the positive control group, Avelumab, is a monoclonal antibody with a bivalent anti-PD-L1 arm, whereas the protein complex of CD3 antibody variable region 2-Fc x PD-L1 antibody and the protein complex of CD3 antibody variable region 3-Fc x PD-L1 antibody are each a multivalent antibody with a monovalent anti-PD-L1 arm. These differences result in significant differences in the level of binding strength.

**[Table 4]**

| Ligand | Experimental group | Binding affinity (KD, M) |
|---|---|---|
| hCD3εδ | CD3 (UCHT1)-binding monoclonal antibody | 1.41 x 10⁻¹¹ |
| | Protein complex of CD3-binding antibody variable region 2-Fc x PD-L1 antibody | 3.41 x 10⁻⁶ |
| | Protein complex of CD3-binding antibody variable region 3-Fc x PD-L1 antibody | 1.04 x 10⁻⁶ |
| hCD3εγ | CD3 (UCHT1)-binding monoclonal antibody | 2.72 x 10⁻¹⁰ |
| | Protein complex of CD3-binding antibody variable region 2-Fc x PD-L1 antibody | 8.15 x 10⁻⁶ |
| | Protein complex of CD3-binding antibody variable region 3-Fc x PD-L1 antibody | 5.63 x 10⁻⁶ |

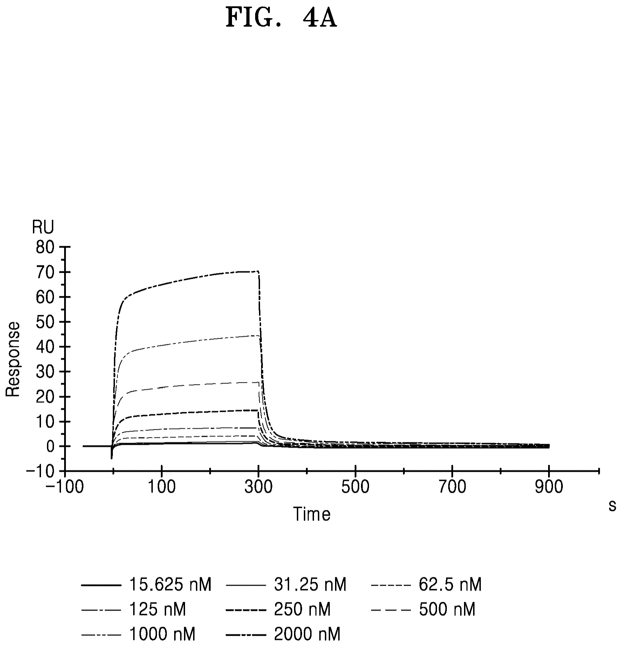

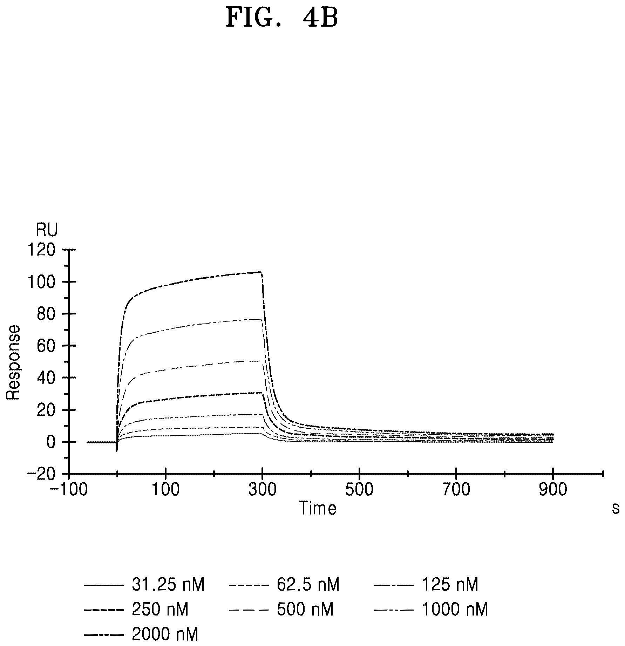

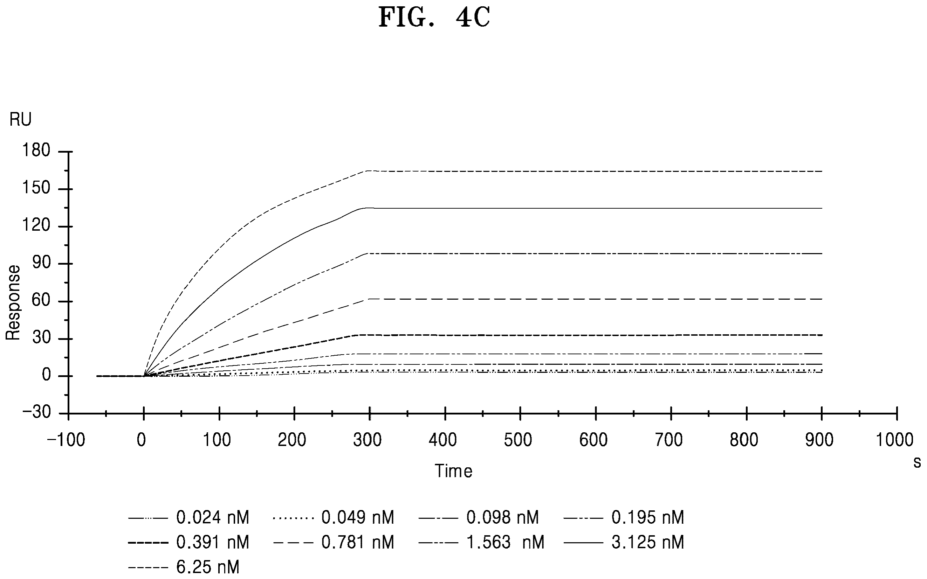

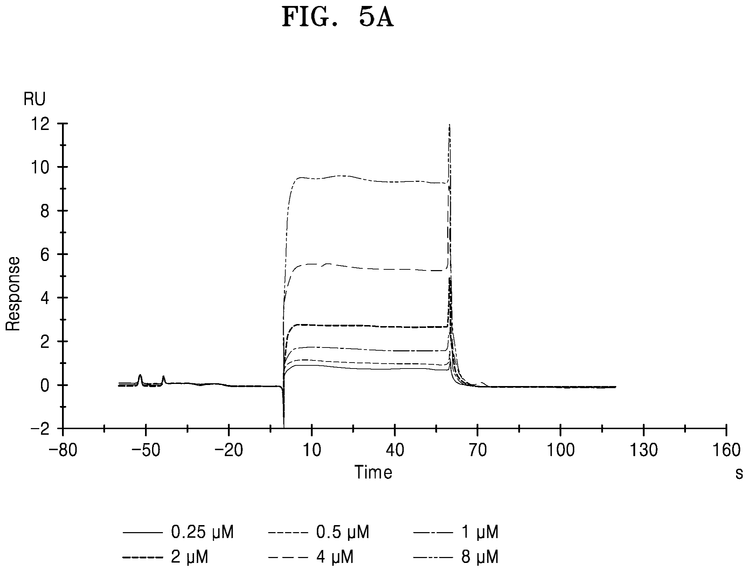

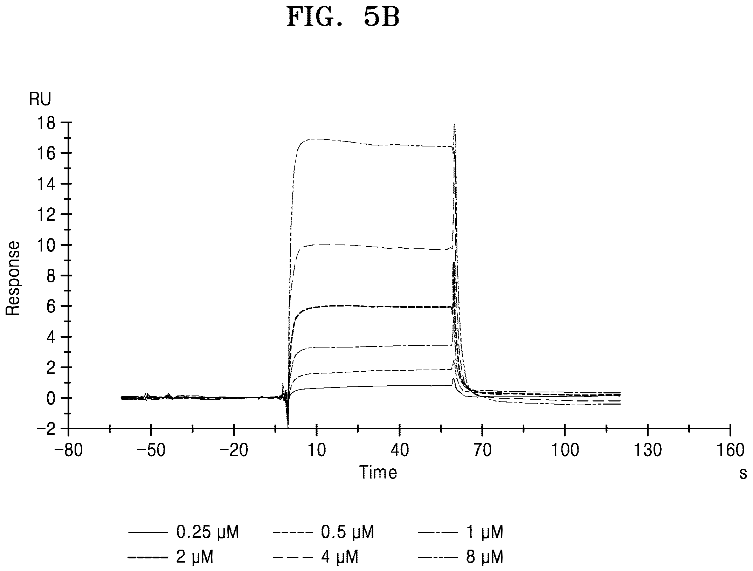

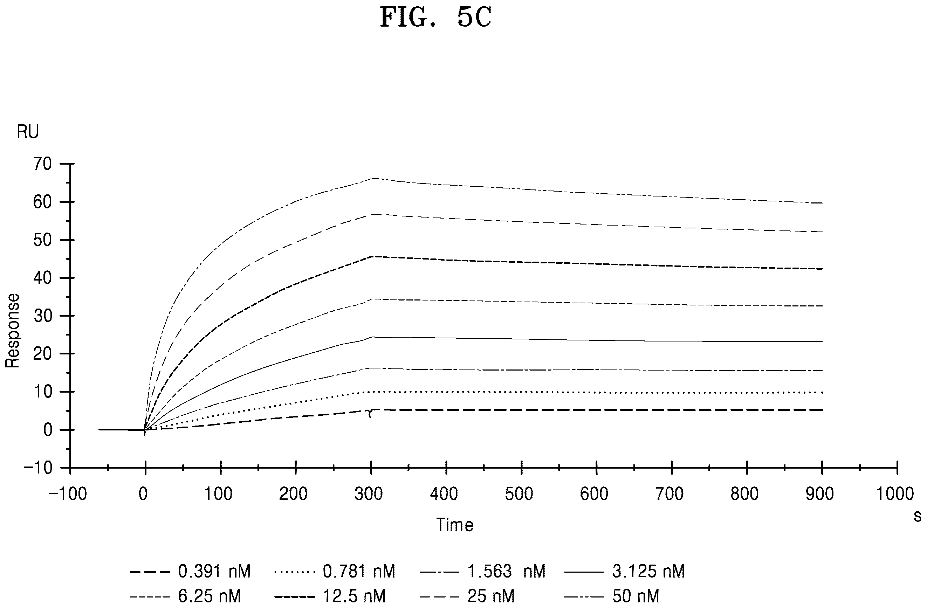

FIGS. 4A to 4C show the results of identifying binding strengths of the protein complex of CD3-binding antibody variable region 2-Fc x PD-L1 antibody, the protein complex of CD3-binding antibody variable region 3-Fc x PD-L1 antibody, and the CD3 (UCHT1)-binding monoclonal antibody, to CD3εδ. FIGS. 5A to 5C show the results of identifying binding strengths of the protein complex of CD3-binding antibody variable region 2-Fc x PD-L1 antibody, the protein complex of CD3-binding antibody variable region 3-Fc x PD-L1 antibody, and the CD3 (UCHT1)-binding monoclonal antibody, to CD3εγ. As a results, as shown in FIGS. 4A to 4C and Table 3, the protein complex of CD3-binding antibody variable region 2-Fc x PD-L1 antibody and the protein complex of CD3-binding antibody variable region 3-Fc x PD-L1 antibody prepared in Example 2 bind to the human CD3εδ with the binding affinities of 3.41 µM and 1.04 µM, respectively, showing a 2.4x10⁵-fold decrease and a 0.7x10⁵-fold decrease compared to the binding strength of the CD3-binding monoclonal antibody (UCHT1) of 0.014 nM.

In addition, as shown in FIGS. 5A to 5C, regarding human CD3εγ, the protein complex of CD3-binding antibody variable region 2-Fc x PD-L1 antibody and the protein complex of CD3-binding antibody variable region 3-Fc x PD-L1 antibody showed the binding affinities of 8.15 µM and 5.63 µM, respectively, showing a 2.9x10⁴-fold decrease and a 2.06x10⁴-fold decrease compared to the binding strength of the CD3 (UCHT1)-binding monoclonal antibody to the human CD3εγ of 0.272 nM.

The binding strengths of the protein complex of CD3-binding antibody variable region 2-Fc x PD-L1 antibody and the protein complex of CD3-binding antibody variable region 3-Fc x PD-L1 antibody, to the CD3εδ were significantly decreased compared to the binding strengths of the same protein complexes to the CD3εγ. Accordingly, although a ratio of the binding strength to CD3εδ / binding strength to CD3εγ was 19.3-fold for the CD3 (UCHT1)-binding monoclonal antibody, it was decreased by 2.4-fold and 5.4-fold for the protein complexes, showing a significant decreased in the binding strength to the CD3εδ.

Referring to the results above, it was confirmed that the binding to CD3 could be significantly decreased by the protein complex of CD3-binding antibody variable region 2-Fc x PD-L1 antibody and the protein complex of CD3-binding antibody variable region 3-Fc x PD-L1 antibody. Also, through this, it is expected that the activation of T cells is inhibited, which may lead to less secretion of cytokines.

### 1-4. Identification of death of cancer cells and level of cytokines released induced by protein complex of CD3-binding antibody variable region-Fc x PD-L1 antibody

Human peripheral blood mononuclear cell-mediated effects on death of cancer cells and release of cytokines induced by the protein complex of CD3-binding antibody variable region 1-Fc x PD-L1 antibody to the protein complex of CD3-binding antibody variable region 7-Fc x PD-L1 antibody were confirmed. Human breast cancer cell line, MDA-MB-231 (ATCC, US), was used as target cancer cells, and human peripheral blood mononuclear cells (Stemcell technologies, CA) were used as effector cells. After co-culturing these cells, the death of the cancer cells was evaluated using flow cytometry, and the release level of inflammatory cytokines were evaluated using ELISA and cytometric bead array. For use as positive groups for these analysis, a protein complex of CD3 (OKT3)-binding antibody variable region-Fc x PD-L1, which has the same structure as the aforementioned protein complex and includes OKT3 clones having high CD3 binding affinity, and a CD3 (UCHT1)-binding monoclonal antibody were used.

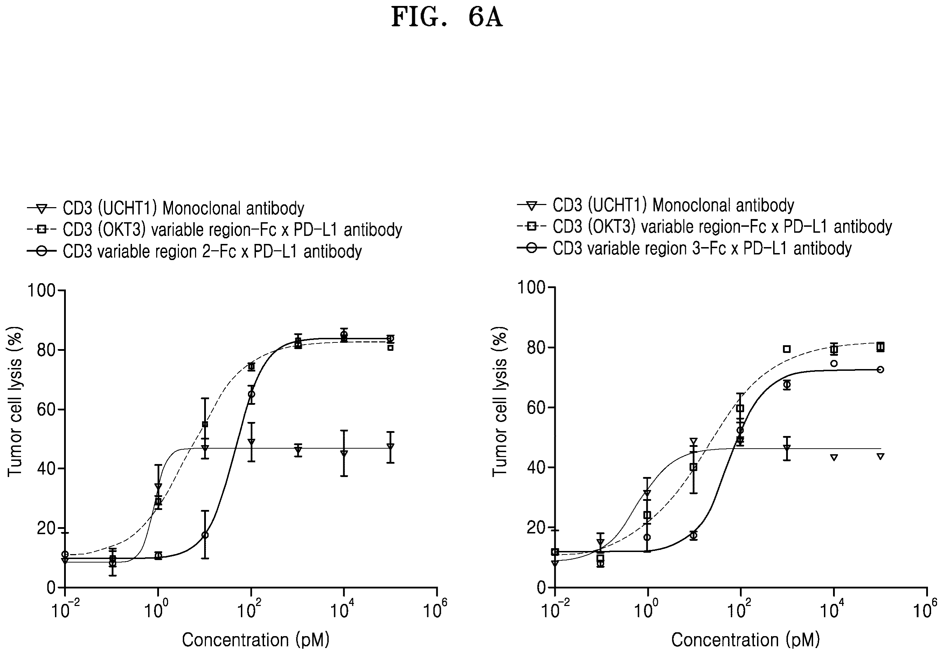

First, to distinguish MDA-MB-231 cells from peripheral blood mononuclear cells, MDA-MB-231 cells were treated with Cell Trace Violet Cell Proliferation ki (Invitrogen, US) diluted 2mM in PBS, and reacted in an incubator at 37 °C for 10 minutes for fluorescence labeling. The labeled cells were seeded at 1 x 10⁴ cells/well in a 96-well plate and cultured for at least 16 hours to prepare target cells. Human peripheral blood mononuclear cells in a frozen state were thawed within 2 minutes in a 37 °C water bath, mixed with RPMI-1640 medium supplemented with 10% FBS, and centrifuged to prepare effector cells. The target cancer cells were treated with the protein complex of CD3-binding antibody variable region-Fc x PD-L1 antibody at various concentrations (10-fold serial dilution within a concentration range of 100 nM to 0.01 pM), and the prepared peripheral blood mononuclear cells were inoculated thereto at 20 times the number of the target cells and co-cultured for 48 hours. To confirm the cancer cell-killing effect on the target cancer cells, after co-culture, the target cells were separated using Accutase (BD, US), washed, and then reacted with 7-AAD (Thermofisher, US) at 4 °C for 30 minutes in the dark to label dead cells. Subsequently, flow cytometry was performed using FACSLyric flow cytometry (BD). The dead cancer cells were distinguished as cell trace violet positive and 7-AAD positive. The results are shown in Table 5 and FIG. 6A.

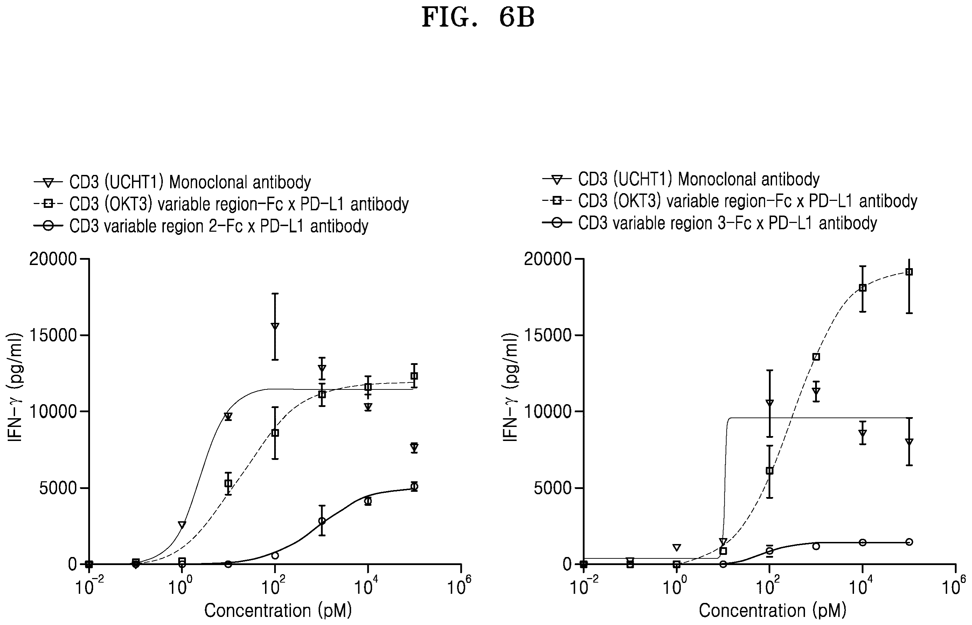

To confirm the cytokine release effect, after co-culture, the concentrations of a specific cytokine in the collected culture medium was measured. The concentration of IFN-γ was measured using human interferon gamma ELISA kit (Abcam, UK). The results are shown in FIG. 6B and Table 5.

The test results confirmed that the maximum cytokine release level was reduced in all test substances compared to the positive control groups.

Compared to the positive control group, i.e., the CD3-binding monoclonal antibody, the protein complex of CD3-binding antibody variable region 1-Fc x PD-L1 antibody to the protein complex of CD3-inding antibody variable region 7-Fc x PD-L1 antibody reduced the maximum cytokine release level by 1.5-fold to 10.5-fold. In particular, the protein complex of CD3-binding antibody variable region 3-Fc x PD-L1 antibody showed a significant reduction by 10.5-fold.

Regarding the tumor cell-killing effects, compared to the CD3 (UCHT1)-binding monoclonal antibody, the protein complex of CD3-binding antibody variable region 1-Fc x PD-L1 antibody to CD3-binding antibody variable region 7-Fc x PD-L1 antibody showed a 1.4-fold to 1.8-fold increase in the maximum cancer cell-killing effect.

In addition, compared to the CD3 (OKT3)-binding double antibody having the same structure as the aforementioned protein complex, the protein complex of CD3-binding antibody variable region 1-Fc x PD-L1 antibody to the protein complex of CD3-binding antibody variable region 7-Fc x PD-L1 antibody showed similar levels of the maximum cancer cell-killing effect, confirming that the cancer cell-killing effect has been maintained.

This indicates that the protein complex of CD3-binding antibody variable region-Fc x PD-L1 antibody in which the binding strength to CD3 has been regulated reduces the binding strength to CD3, while maintaining tumor lysis effects similar to those of the positive control groups, thereby reducing the maximum level of cytokine release induced by the death of the cancer cells, confirming a significant level of safety.

**[Table 5]**

| Name of substance | Death of cancer cells | | IFNγ release | |
|---|---|---|---|---|
| | EC₅₀ nM | Emax (%) | EC₅₀ nM | Maximum concentration (ng/ml) |
| CD3 (UCHT1)-binding monoclonal antibody | 0.0005 | 46 | 0.003 | 16 |
| Protein complex of CD3 (OKT3)-binding antibody variable region x PD-L1 antibody | 0.02 | 77 | 0.3 | 19 |
| Protein complex of CD3-binding antibody variable region 1-Fc x PD-L1 antibody | 0.23 | 80 | 0.1 | 6.1 |
| Protein complex of CD3-binding antibody variable region 2-Fc x PD-L1 antibody | 0.05 | 85 | 2.5 | 9.8 |
| Protein complex of CD3-binding antibody variable region 3-Fc x PD-L1 antibody | 0.06 | 71 | 0.1 | 1.47 |
| Protein complex of CD3-binding antibody variable region 4-Fc x PD-L1 antibody | 0.18 | 79 | 31.2 | 2.6 |
| Protein complex of CD3-binding antibody variable region 5-Fc x PD-L1 antibody | 0.18 | 66 | 10.0 | 4.4 |
| Protein complex of CD3-binding antibody variable region 6-Fc x PD-L1 antibody | 3.50 | 68 | 16.4 | 5.1 |
| Protein complex of CD3-binding antibody variable region 7-Fc x PD-L1 antibody | 2.26 | 68 | 15.3 | 9.3 |

### 1-5. Identification of cytokine release by protein complex of CD3-binding antibody variable region-Fc x PD-L1 antibody depending on presence or absence of cancer cells

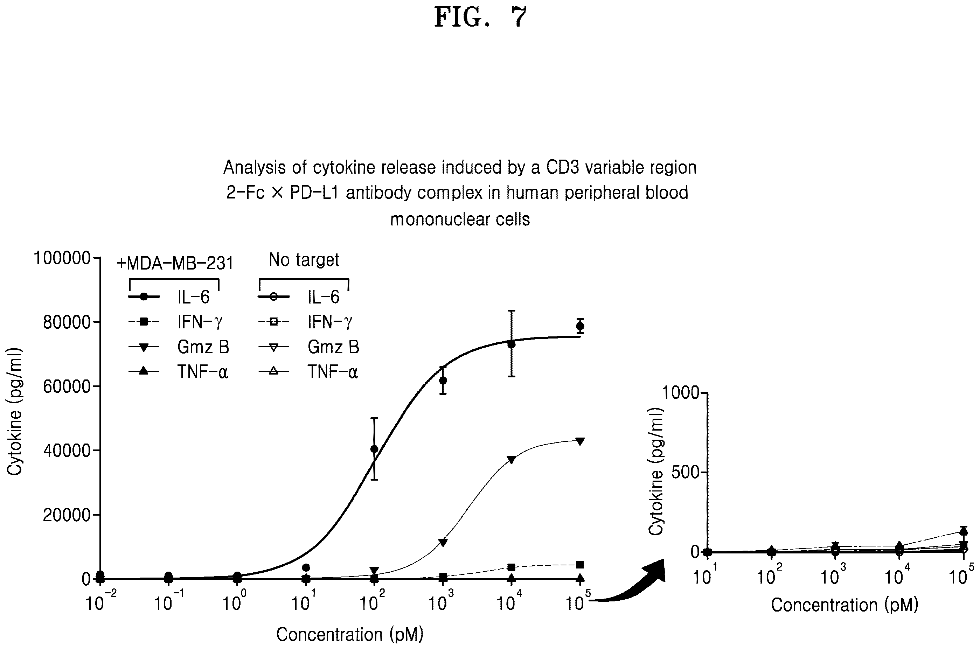

Human peripheral blood mononuclear cell-mediated effects on cytokine release induced by the protein complex of CD3-binding antibody variable region 2-Fc x PD-L1 antibody in the presence or absence of cancer cells were confirmed. First, human breast cancer cell line, MDA-MB-231 (ATCC, US), as target cancer cells were seeded at 1x10⁴ cells/well in a 96-well plate and cultured for at least 16 hours to prepare target cells. Human peripheral blood mononuclear cells (Stemcell technologies, CA) in a frozen state were thawed within 2 minutes in a 37 °C water bath, mixed with RPMI-1640 medium supplemented with 10% FBS, and centrifuged to prepare effector cells. A 96-well plate containing the previously prepared target cancer cells or not containing target cancer cells was treated with the protein complex of CD3-binding antibody variable region 2-Fc x PD-L1 antibody at various concentrations (10-fold serial dilution within a concentration range of 100 nM to 0.01 pM), and the prepared peripheral blood mononuclear cells were inoculated thereto at 10 times the number of the target cells and co-cultured for 48 hours. After co-culture, the concentrations of specific cytokines, such as IFN-γ, IL-6, TNF-α, and Granzyme B, in the collected culture medium were measured using cytometric bead array (BD) and FACSLyric flow cytometry (BD), and then analyzed using CBA analysis software (BD). The results are shown in FIG. 7 and Table 6.

**[Table 6]**

| Maximum concentration (ng/mL) | MDA-MB-231 + human peripheral blood mononuclear cells | Human peripheral blood mononuclear cells |
|---|---|---|
| IL-6 | 79 | 0.021 |
| IFN | 4 | 0.037 |
| Gmz B | 43 | 0.057 |
| TNF | 0.1 | 0.027 |

As a result, it was confirmed that the protein complex of CD3-binding antibody variable region 2-Fc x PD-L1 antibody released cytokines when the effector cells and the target cells were co-cultured. However, when only the effector cells were cultured without the target cells, the ability to induce the cytokine release was found to be significantly reduced.

### 1-6. Identification of cell-killing levels in cancer cells and normal cells by protein complex of CD3-binding antibody variable region-Fc x PD-L1 antibody

Human peripheral blood mononuclear cell-mediated effects on death of cancer cells and normal cells by the protein complex of CD3-binding antibody variable region 2-Fc x PD-L1 antibody were confirmed. For use as target cancer cells, human breast cancer cell lines, i.e., MDA-MB-231 (ATCC, US) with high PD-L1 expression and MCF-7 (ATCC, US) with low PD-L1 expression, were used and co-cultured with normal cells with low PD-L1 expression, i.e., 293T (ATCC, US) and human peripheral blood mononuclear cells (Stemcell technologies, CA). Afterwards, the cell-killing effect was evaluated using flow cytometry. For use as a positive group for the analysis, a CD3 (UCHT1)-binding monoclonal antibody (Biolegend, US), Avelumab (Pfizer, US), was used.

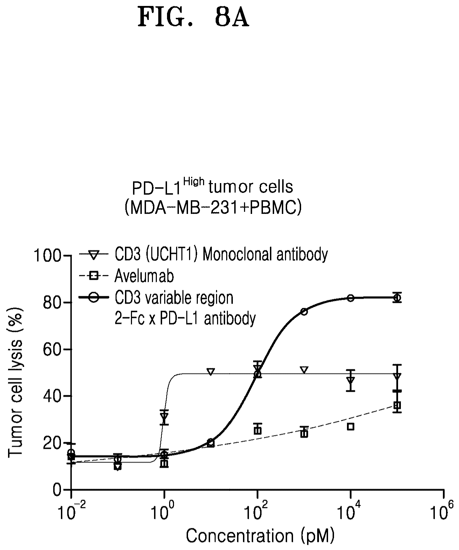

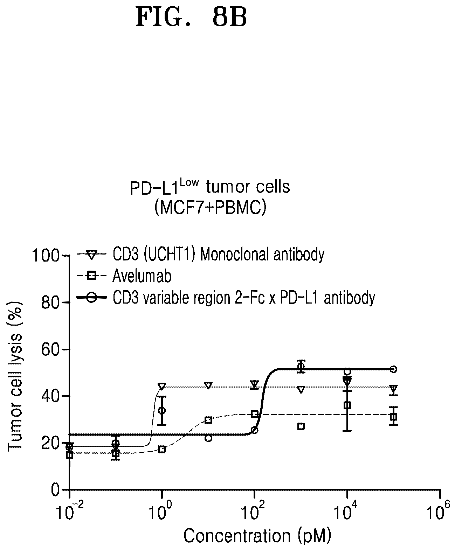

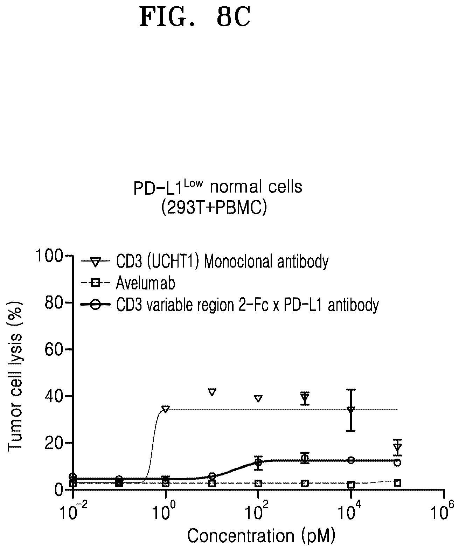

First, to distinguish cancer cells and normal cells from peripheral blood mononuclear cells, the cancer cells and normal cells were treated with the Cell Trace Violet Cell Proliferation Kit (Invitrogen, USA) diluted to 2 mM in PBS, and reacted in an incubator at 37 °C for 10 minutes for fluorescence labeling. The labeled cells were seeded at 1 x 10⁴ cells/well in a 96-well plate and cultured for at least 16 hours to prepare target cells. Human peripheral blood mononuclear cells in a frozen state were thawed within 2 minutes in a 37 °C water bath, mixed with RPMI-1640 medium supplemented with 10% FBS, and prepared by centrifugation. The target cancer cells were treated with the protein complex of CD3-binding antibody variable region 2-Fc x PD-L1 antibody at various concentrations (10-fold serial dilution within a concentration range of 100 nM to 0.01 pM), and the prepared peripheral blood mononuclear cells were inoculated thereto at 20 times the number of the cancer cells and 10 times the number of the normal cells and co-cultured for 48 hours. To confirm the target cell-killing effect, after co-culture, the target cells were detached using Accutase (BD, US), washed, and then reacted with 7-AAD (Thermofisher, US) at 4 °C for 30 minutes in the dart to label dead cells. Subsequently, flow cytometry was performed using FACSLyric flow cytometry (BD). The dead cells were classified as cell trace violet positive and 7-AAD positive. The results are shown in FIGS. 8A to 8C and Table 7.

**[Table 7]**

| Tumor lysis | EC50(pM) | Emax (%) |
|---|---|---|
| MDA-MB-231 | 94.5 | 82 |
| MCF7 | 150.2 | 51 |
| 293T | 31.3 | 13 |

As a result, it was confirmed that the cell-killing extent of the protein complex of CD3binding antibody variable region 2-Fc x PD-L1 antibody differed depending on the degree of PD-L1 expression in the target cells. The cell-killing effect was found to be induced by which the MDA-MB-231 which is a cancer cell with high PD-L1 expression showed an 82 % cell-killing effect, whereas the MCF7 which is a cancer cell with low PD-L1 expression showed a 51% cell-killing effect. However, the 293T which is a normal cell line with low PD-L1 expression showed a very low cell-killing level of 13 %. Accordingly, it was confirmed that the protein complex of CD3-binding antibody variable region 2-Fc x PD-L1 antibody induced cell death specifically in cancer cells with highly expressed PD-L1.

### 1-7. Identification of anticancer effect of protein complex of CD3-binding antibody variable region-Fc x PD-L1 antibody in humanized xenograft mice

The anticancer efficacy of the protein complex of CD3-binding antibody variable region 2-Fc x PD-L1 antibody was evaluated in a humanized xenograft model implanted with MDA-MB-231 breast cancer cells that are known to overexpress PD-L1. For use as positive groups for this experiment, Avelumab and a protein complex of CD3 (OKT3)-binding antibody variable region-Fc x PD-L1, which has the same structure as the aforementioned protein complex and includes OKT3 clones having high CD3 binding affinity, were used.

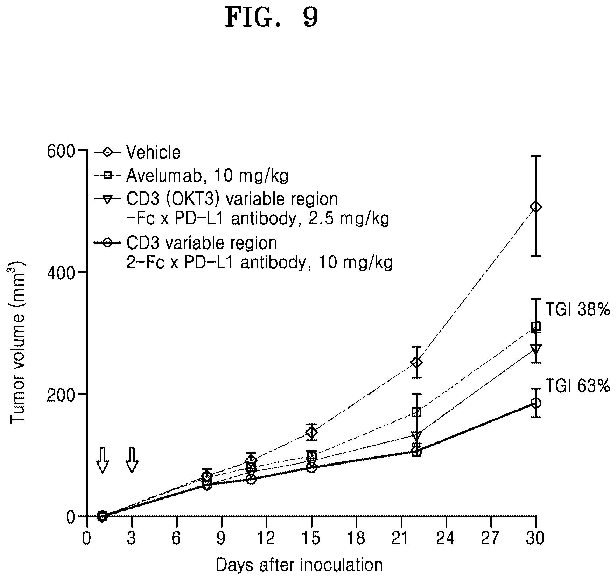

First, 7-week-old female NOD/SCID mice were supplied by KOATECH Co. Ltd. and acclimatized for one week before use in the test. To induce the human immune system, human peripheral blood mononuclear cells (Stemcell technologies, CA) stored in frozen form were rapidly thawed, and only CD3-positive T cells were separated using CD3 microbeads (Miltenyi biotech). For each mouse, 2.5 x 10⁶ CD3-positive T cells and 5.0 x 10⁶ MDA-MB-231 (ATCC, US) cells for tumor production were mixed at a volume of 1:1, and the mixture was mixed with Matrigel at a volume ratio of 1:1 and injected subcutaneously. On Days 0, 2, and 4 after administration of the cancer cells and CD3-positive T cells, the PD-L1 antibody (Avelumab), the protein complex of CD3 (OKT3)-binding antibody variable region-Fc x PD-L1 antibody, and the protein complex of CD3-binding antibody variable region 2-Fc x PD-L1 antibody in which the binding affinity to CD3 has been regulated were administered intravenously at doses of 10, 2.5, 10 mg/kg, respectively. The tumor growth was observed for 30 days after the first drug administration, and the results are shown in FIG. 9.

As a result, as shown in FIG. 9, in the humanized xenograft animal model, the vehicle caused an increase in tumor volume over time, whereas the protein complex of CD3-binding antibody variable region 2-Fc x PD-L1 antibody demonstrated a 63 % reduction in tumor volume by Day 30, showing superior anticancer efficacy to a 38 % reduction observed in the group administered with Avelumab alone.

This demonstrated excellent anticancer effects of the protein complex of CD3-binding antibody variable region 2-Fc x PD-L1 antibody through synergistic effects by multiple actions on the human tumor targets and human T cell activation, as compared to the therapeutic effects of anti-PD-L1 monotherapy.

### 1-8. Identification of anticancer effect of protein complex of CD3-binding antibody variable region-Fc x PD-L1 antibody in hCD3 Knock-in mice

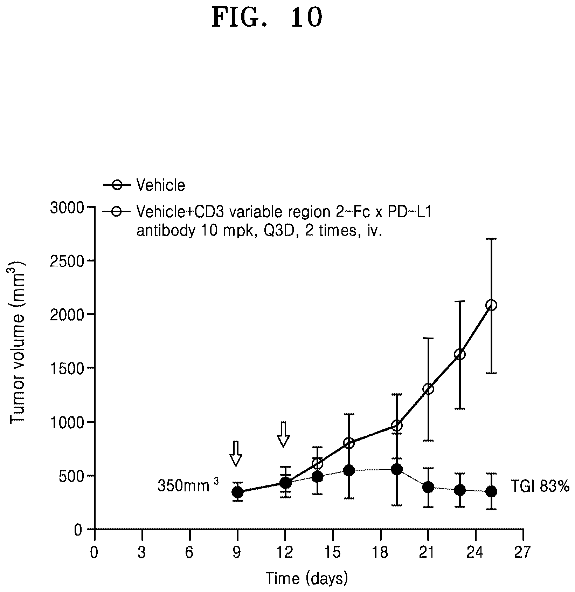

To evaluate the anticancer efficacy of the protein complex of CD3-binding antibody variable region 2-Fc x PD-L1 antibody in an animal model with an intact immune system, the efficacy of human CD3-binding antibody drugs was evaluated in a CT26 colorectal cancer-induced animal model using human CD3 knock-in mice (Balb/c) that were responsive to the drugs. First, CT26 colorectal cancer cells were subcutaneously administered to 7-week-old experimental animals at a concentration of 1.0 x 10⁶/100 uL to induce tumor production. Following the induction of tumor production, the animals were divided into groups of four when tumor volume reached an average of 70 mm². From Day 9 at which tumor volume reached an average of 350 mm², the drug was administered intravenously once every three days, for a total of two times. The level of tumor growth induced by the drug administration was observed until Day 27 after animal group classification, and the results are shown in FIG. 10.

As a result, it was confirmed that the protein complex of CD3-binding antibody variable region 2-Fc x PD-L1 antibody reduced the tumor volume by 83 % compared to the vehicle in the PD-L1-expressing cancer cells.

This also demonstrated excellent anticancer effect without side effects in a model using the immune system of mice expressing human CD3. Therefore, the protein complex of CD3-binding antibody variable region 2-Fc x PD-L1 antibody in which the binding affinity has been regulated can provide a safer therapeutic agent by reducing side effects caused by excessive release of pro-inflammatory cytokines observed in existing CD3-binding antibodies, while maintaining excellent anticancer effect.

### 1-9. Identification of cytokine release levels inhibited by protein complex of CD3-binding antibody variable region-Fc x PD-L1 antibody in hCD3 knock-in mice

To evaluate the extent of cytokine release induced by the protein complex of CD3-binding antibody variable region 2-Fc x PD-L1 antibody in which the binding affinity to CD3 has been regulated in an animal model with an intact immune system, anti-human CD3 drugs were administered to a CT26 colorectal cancer-induced animal model using human CD3 knock-in mice (Balb/c) that were responsive to the drugs, and then the concentrations of pro-inflammatory cytokines, such as IL-6, IFN-γ, and TNF-α, in blood were analyzed as follows. For use as a positive group for the analysis, a protein complex of CD3 (OKT3)-binding antibody variable region-Fc x PD-L1, which has the same structure as the aforementioned protein complex and includes OKT3 clones having high CD3 binding affinity, was used.

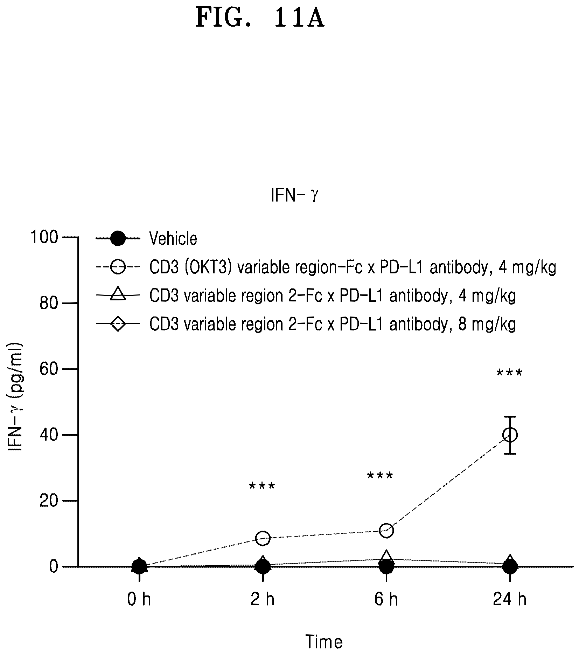

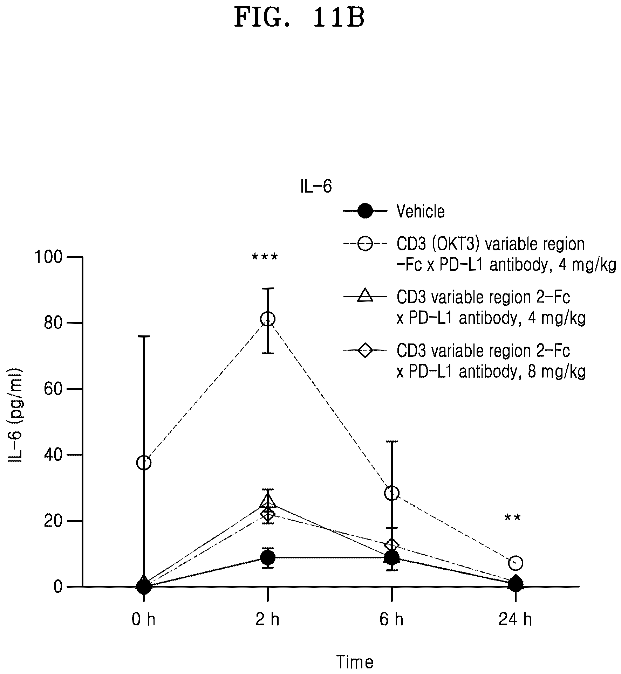

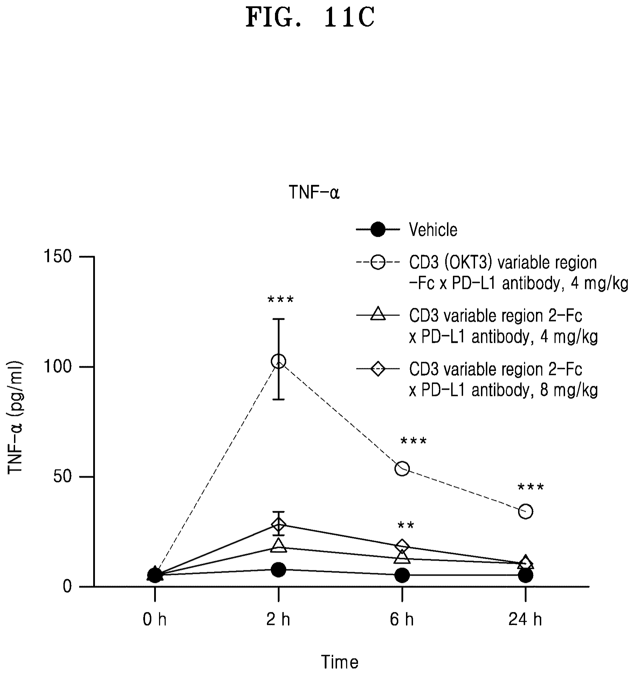

First, CT26 colorectal cancer cells were subcutaneously administered to 7-week-old experimental animals at a concentration of 1.0 x 10⁶/100 uL to induce tumor production. Following the induction of tumor production, the animals were divided into groups of six when tumor volume reached an average of 70 mm², and a single dose of 4 mg/kg of the positive control group and a single dose of 4 and 8 mg/kg of the protein complex of CD3-binding antibody variable region 2-Fc x PD-L1 antibody in which the binding affinity to CD3 has been regulated were administered intravenously. Within 24 hours after the administration, blood samples were collected from three animals at the points of 0 hour, 2 hours, 6 hours, and 24 hours to measure cytokine release levels, and 50 µL of serum was obtained from each animal to analyze cytokine concentrations in serum at each measurement time point using BD's cytometric bead array technique. The statistical significance was verified using Dunnett's multiple comparison test, and the results are shown in Table 8 and FIGS. 11A to 11C (** p < 0.01, *** p < 0.001).

The results of measuring the cytokine release levels over time showed that the cytokine release level by the protein complex of CD3-binding antibody variable region 2-Fc x PD-L1 antibody was significantly lower than that of the positive control group in which binding affinity has not been regulated. The maximum release level of IFN-γ in the positive control group was 51.7 pg/mL at the maximum concentration at 24 hours, whereas, in the group administered with 4 and 8 mpk of the protein complex of CD3-binding antibody variable region 2-Fc x PD-L1 antibody, the maximum release level of IFN-γ was 3.4 and 3.7 pg/mL, respectively, at the maximum concentration at 6 hours. These results show that the maximum release levels were reduced by 15.2-fold and 14-fold, respectively, compared to the positive control group.

For the release level of IL-6, the positive control group showed a maximum release level of 114.3 pg/mL at 6 hours, whereas the groups administered with 4 and 8 mpk of the protein complex of CD3-binding antibody variable region 2-Fc x PD-L1 antibody showed maximum release levels of 31.3 pg/mL and 25.5 pg/mL, respectively at 2 hours. These results show that the release levels were reduced by 3.7-fold and 4.5-fold compared to the maximum release level of the positive control group.

The release levels of TNF-α were found to reach the maximum at 2 hours in both the positive control group and the protein complex of CD3-binding antibody variable region 2-Fc x PD-L1 antibody, wherein the positive control group showed the maximum release level of 138.6 pg/mL and the groups administered with 4 mpk and 8 mpk of the protein complex of CD3-binding antibody variable region 2-Fc x PD-L1 antibody, respectively, showed the maximum release levels of 19.9 pg/mL and 38.8 pg/mL, respectively, indicating that the maximum release levels were reduced by 7-fold and 3.6-fold, respectively, compared to the positive control group.

The positive control group with high binding affinity to CD3 showed significantly higher release levels of all cytokines compared to the protein complex of CD3-binding antibody variable region 2-Fc x PD-L1 antibody, and the protein complex of CD3-binding antibody variable region 2-Fc x PD-L1 antibody in which the binding affinity to CD3 has been regulated induced relatively very low levels of cytokine release, suggesting that adverse effects would be minimized.

**[Table 8]**

| **Maximum concentration (pg/mL)** | **IFNγ** | **IL-6** | **TNFα** |
|---|---|---|---|
| CD3(OKT3) variable region-Fc x PD-L1, 4 mpk | 51.7 (24 h) | 114.3 (6 h) | 138.6 (2 h) |
| CD3 variable region 2-Fc x PD-L1, 4 mpk | 3.4 (6 h) | 31.3 (2 h) | 19.9 (2 h) |
| CD3 variable region 2-Fc x PD-L1, 8 mpk | 3.7 (6 h) | 25.5 (2 h) | 38.8 (2 h) |

### Example 2. Preparation and confirmation of activity level of protein complex of CD3-binding antibody variable region-Fc x GPC3 antibody

### 2-1. Cloning and culturing of protein complex of CD3-binding antibody variable region-Fc x GPC3 antibody

A protein complex including a CD3-binding antibody variable region -Fc and a GPC3 antibody was prepared. Specifically, a protein complex including anti-GPC3 Fab-Fc and CD3-binding antibody variable region-F was obtained using the same method as in Example 1-1, except that a polynucleotide encoding the heavy chain variable region-constant region of the GPC3 and a polynucleotide encoding the light chain variable region-constant region of the GPC3 were used.

### 2-2. Purification of protein complex of CD3-binding antibody variable region-Fc x GPC3 antibody

To obtain high-purity substances of the protein complex of CD3-binding antibody variable region-Fc x GPC3 antibody obtained in Example 2-1, high-purity substances were obtained by fast protein liquid chromatography (FPLC, Cytiva) using Protein A affinity column and hydrophobic interaction column. First, the culture medium was centrifuged to separate the cultured cells from the medium, and the protein complex of CD3-binding antibody variable region-Fc x GPC3 antibody in the separated medium was filtered through a 0.22 µm filter (Thermo Scientific) to remove fine debris. The resulting medium from the filtration process was then subjected to first purification using Protein A affinity chromatography (MabSelect PrismA, Cytiva), followed by second purification using Phenyl Sepharose High Performance (Phenyl HP) chromatography (Cytiva) based on hydrophobic interactions to obtain high-purity substances. The above processes can be explained in detail as follows.

The Protein A column stabilized with phosphate buffer saline (pH 7.4) was filled with the medium resulting from the filtration process, and non-specifically bound proteins were washed off with the same buffer, and washed off again with buffer containing 50 mM acetic acid + 1 M sodium chloride (pH 5.5). To remove additional impurities, the sample was finally washed with a 200 mM acetic acid (pH 5.5) buffer solution. Afterwards, proteins bound specifically to the Protein A column were eluted using a 20 mM acetic acid buffer solution (pH 5.0 and pH 3.5) according to a pH descending gradient method. The eluted proteins were then used to neutralize the sample to pH 7.2 using 1 M Tris buffer. Subsequently, the second purification intended to remove impurities derived from residual substances in the sample separated from the affinity column used hydrophobic interaction column (Phenyl HP, Cytiva), according to the following procedures.

First, the sample eluted and concentrated in the Protein A column process was added to the Phenyl HP column stabilized with 20 mM sodium phosphate (pH 7.0) buffer and 800 mM ammonium sulfate (pH 7.0), and 1 M citric acid was added thereto to adjust the pH to pH 7.0. Then, non-specifically bound proteins were washed off with the same buffer, and by elution using a buffer solution containing 20 mM sodium phosphate (pH 7.0) according to a descending gradient method for salt concentrations, protein complexes of GPC3 antibody-CD3 antibody protein complex were obtained. The obtained protein complexes of GPC3 antibody-CD3 antibody were stabilized with phosphate buffer saline (pH 6.0). Purity analysis was performed using size exclusion HPLC (TSK-3000SWxL, 7.8 mm x 30 cm, Tosoh) with antibodies that had undergone all of the above purification processes.

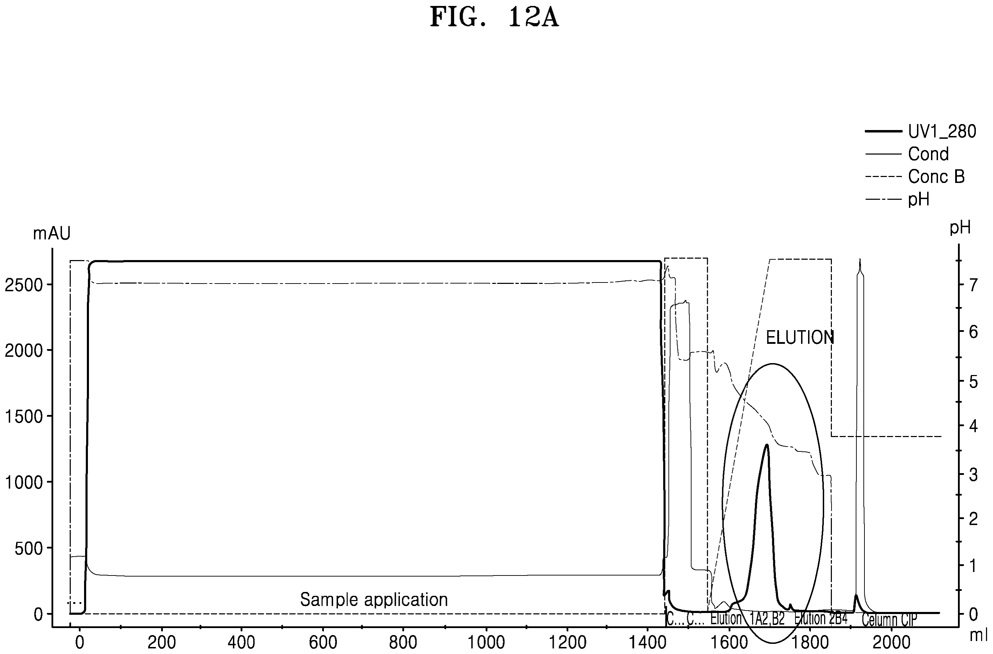

FIG. 12A shows the results of purification of the protein complex of CD3-binding antibody variable region 3-Fc x GPC3 antibody, by using the Protein A affinity column purification method.

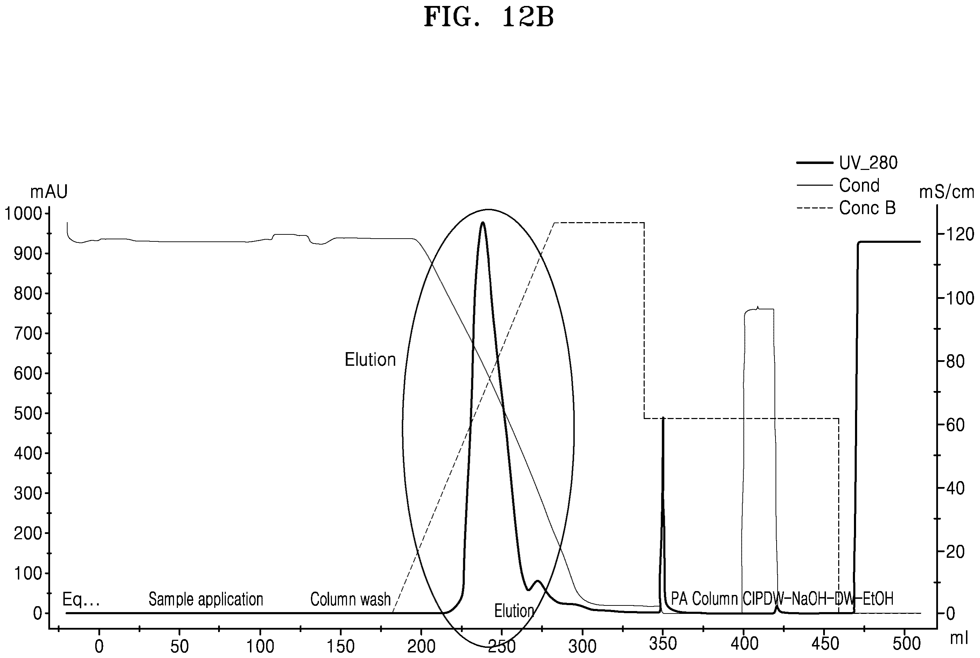

FIG. 12B shows the results of purification of the protein complex of CD3-binding antibody variable region 3-Fc x GPC3 antibody, by using the Phenyl HP hydrophobic interaction column purification method.

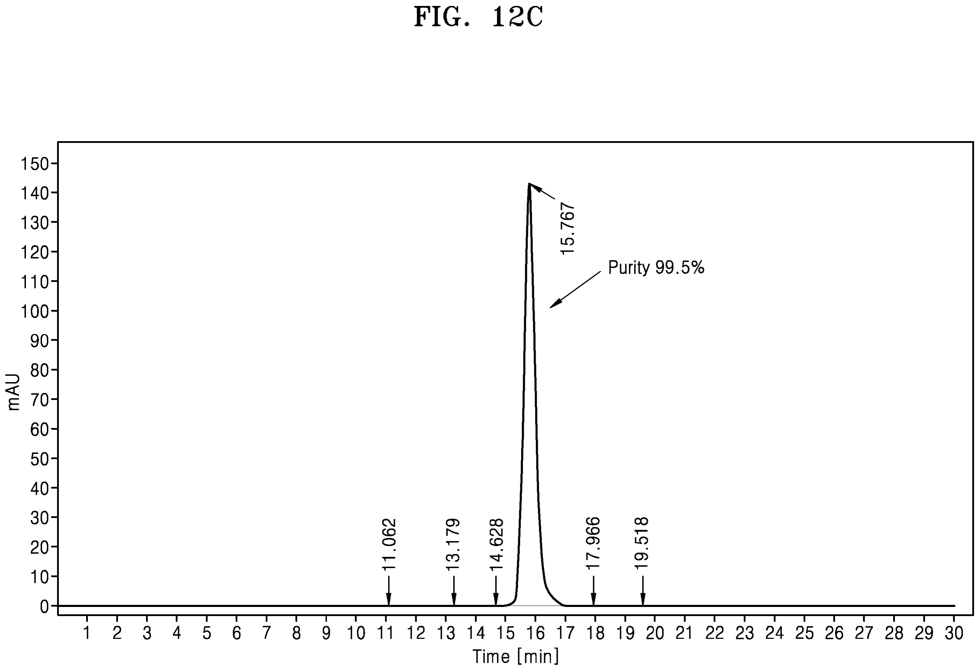

FIG. 12C shows the results of purity analysis of the protein complex of CD3-binding antibody variable region 3-Fc x GPC3, antibody, according to an aspect.

As a result, as shown in FIG. 12A, proteins bound specifically to the column were confirmed using the elution buffer used in the Protein A affinity column. Specifically, the protein complex of CD3-binding antibody variable region-Fc x GPC3 antibody bound specifically to the column was also confirmed in 1,600 to 1,750 ml of the eluate.

As a result, as shown in FIG. 12B, impurities remaining in the eluate used in the Protein A affinity column and non-specifically bound proteins were confirmed.

In addition, as shown in FIG. 12C, it was confirmed that the protein complex of CD3-binding antibody variable region 3-Fc x GPC3 antibody obtained by the Protein A affinity column purification and cation-exchange resin purification showed a purity of 99.5 % at a retention time of 15.767 minutes during purity analysis using size exclusion column.

That is, it was confirmed that the protein complex of CD3-binding antibody variable region-Fc x GPC3 antibody, according to an aspect, was purified with high purity.

### 2-3. Identification of binding strength of protein complex of CD3-binding antibody variable region-Fc x GPC3 antibody

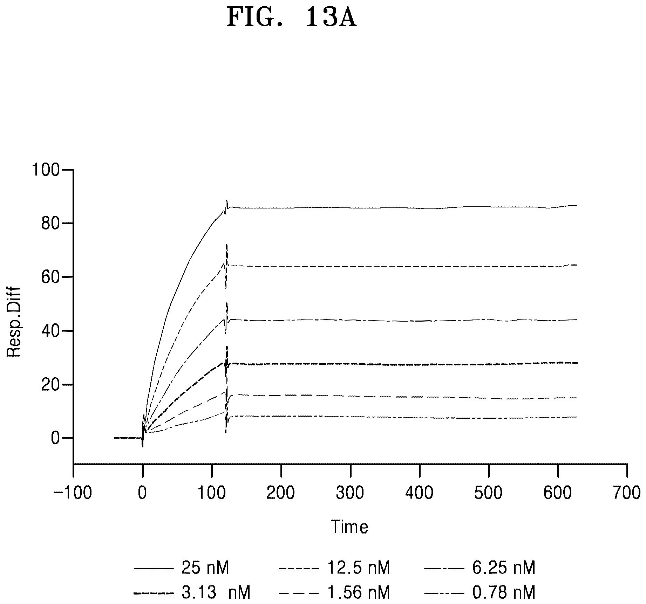

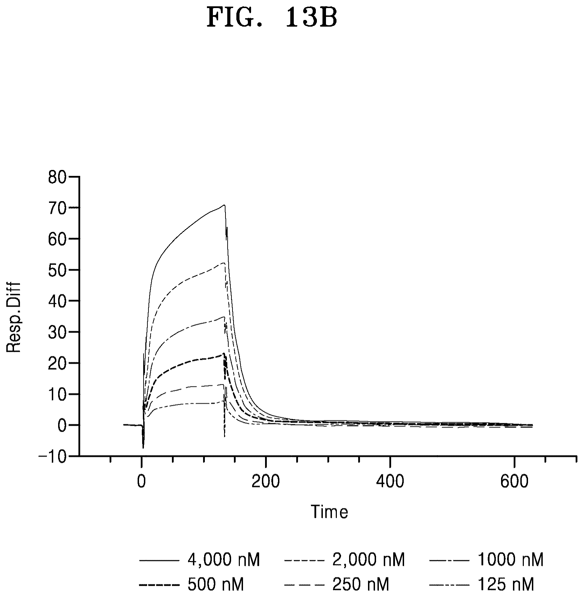

The binding strength of the protein complex of CD3-binding antibody variable region 3-Fc x GPC3 antibody, according to an aspect, to human CD3 receptors was confirmed. The binding strength was evaluated by surface plasmon response (SPR) analysis using Biacore T200 (Cytiva, Sweden). Specifically, human CD3εδ was immobilized on a CM5 sensor chip via a covalent bond. Subsequently, the protein complex of CD3-binding antibody variable region 3-Fc x GPC3 antibody was applied at various developing concentrations (125 to 4000 nM) onto the chip to confirm the binding kinetics for the human CD3 receptors. Next, the binding affinity (KD) was calculated using the measured association constant (Ka) and dissociation constant (Kd) values. Here, the surface plasmon resonance (SPR) sensorgram analysis was performed using the BIAlogue kinetics evaluation software. For use as a positive control group, a CD3 (UCHT1)-binding monoclonal antibody was used, and the results are shown in Table 9 and FIGS. 13A and 13B.

**[Table 9]**

| **Ligand** | **Experimental group** | **Binding affinity (KD, M)** |
|---|---|---|
| Human CD3εδ | CD3 (UCHT1)-binding monoclonal antibody | 3.41 x 10⁻¹¹ |
| | Protein complex of CD3 variable region 3 x GPC3 antibody | 3.39 x 10⁻⁶ |

FIG. 13A shows the results of identifying the binding strength of the CD3 (UCHT1)-binding monoclonal antibody to the CD3εδ, and FIG. 13B shows the results of identifying the binding strength of the protein complex of CD3-binding antibody variable region 3-Fc x antibody GPC3 to the CD3ε. As a result, as shown in Table 9, it was confirmed that the protein complex of CD3-binding antibody variable region 3-Fc x GPC3 antibody prepared in Example 2-2 and the positive control group, i.e., the CD3 (UCHT1)-binding monoclonal antibody, bind to the human CD3εδ antigen in a concentration-dependent manner. Specifically, the protein complex showed the binding strength of 3.39 µM and 0.0341 nM, respectively.

This result indicates that the binding strength of the protein complex of CD3-binding antibody variable region 3-Fc x GPC3 antibody to the human CD3εδ was reduced by 1x10⁵-fold compared to the binding strength of the CD3 (UCHT1)-binding monoclonal antibody.

It is found to be similar to the binding strength of 1.04 µM of the protein complex of CD3-binding antibody variable region 3-Fc x PD-L1 antibody to the CD3εδ as confirmed in Example 1-3, indicating that, as a bispecific antibody, the binding strength to the human CD3εδ is maintained even when the target of the arm other than the CD3-targeting arm is changed.

These results suggest that the binding strength of the protein complex of CD3-binding antibody variable region 3-Fc x GPC3 antibody to the CD3εδ is significantly reduced, thereby effectively reducing the binding to CD3 and accordingly inducing the release of cytokines.

### 2-4. Identification of activation level of T cells by GPC3 expression by protein complex of CD3-binding antibody variable region-Fc x GPC3 antibody

The extent of activation of T cells derived from human peripheral blood mononuclear cells by the protein complex of CD3-binding antibody variable region-Fc x GPC3 antibody was confirmed. For use as target cancer cells, human liver cancer cell line Huh-7 (JCRB Cell Bank, Japan) with intermediate GPC3 expression levels and human liver cancer cell line HepG2 (ATCC, US) with high GPC3 expression levels were used, and for effector cells, human peripheral blood mononuclear cells (Stemcell technologies, US) were used and co-cultured. Afterwards, the extent of activation of T cells was analyzed by flow cytometry, and the extent of activation of T cells under conditions without the target cells was analyzed. For use as a positive control group for the analysis, a CD3 (UCHT1)-binding single antibody (Biolegend, US) was used, and the activity of the protein complex of CD3-binding antibody variable region 3-Fc x GPC3 antibody in which the binding affinity to the CD3-binding antibody has been regulated was evaluated.

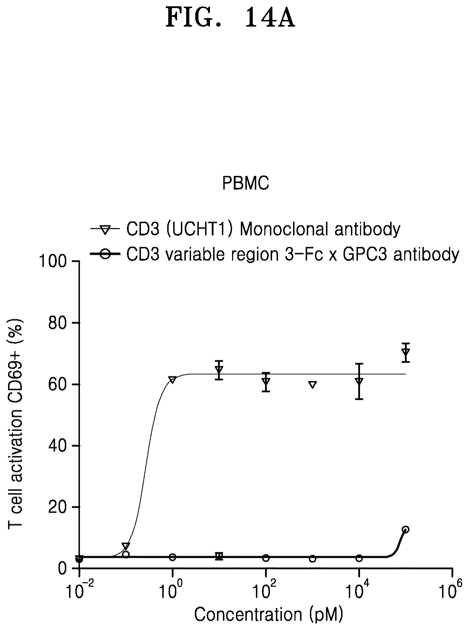

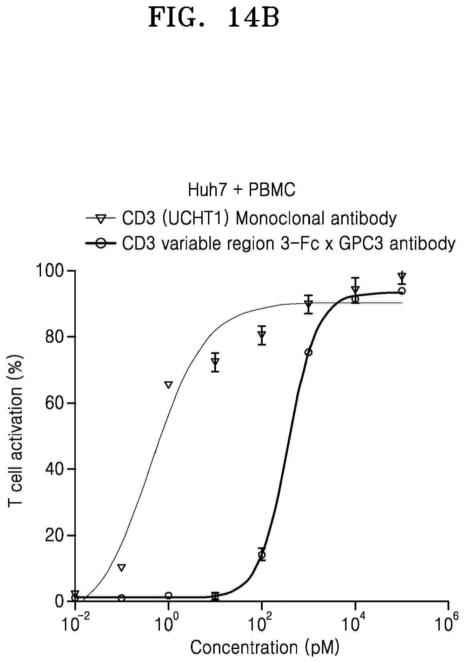

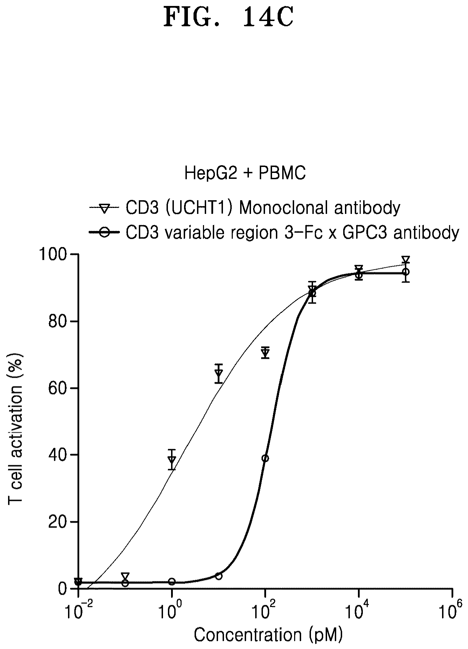

First, to distinguish the cancer cells and the peripheral blood mononuclear cells, the cancer cells were treated using a 2 mM cell trace violet-cell proliferation kit (Invitrogen, US), and reacted in an incubator at 37 °C for 10 minutes for fluorescence labeling. The labeled cells were seeded at 1 x 10⁴ cells/well in a 96-well plate and cultured for at least 16 hours to prepare target cells. Human peripheral blood mononuclear cells in a frozen state were thawed within 2 minutes in a 37 °C water bath, mixed with DMEM medium supplemented with 10% FBS, and centrifuged to prepare effector cells. The target cancer cells were treated with the CD3 monoclonal antibody and the protein complex of CD3-binding antibody variable region 3-Fc x GPC3 antibody at various concentrations (serial dilution by 1/10 within a concentration range of 100 nM to 0.01 pM), and the prepared peripheral blood mononuclear cells were inoculated thereto at 20 times the number of the target cells and co-cultured for 48 hours. Meanwhile, the extent of activation of T cells in the absence of the target cells was additionally analyzed under the same substance treatment conditions. To confirm the extent of activation of T cells, after 48 hours of culture, the target cancer cells were detached using Accutase (BD Bioscience, US), washed with PBS, and reacted with a CD3 antibody, a CD8 antibody, and a CD69 antibody (BD Bioscience, US) at 4 °C in the dark for 30 minutes to label markers for the CD8+ T cells and the activation. Subsequently, flow cytometry was performed using FACSLyric flow cytometry (BD Biosceicne, US). The cancer cells were classified as cell trace violet positive, and the extent of activation of T cells was classified as CD3 positive, CD8 positive, and CD69 positive, and the results are shown in FIGS. 14A to 14C.

As a result, it was confirmed that the extent of cell activation by the protein complex of CD3-binding antibody variable region 3-Fc x GPC3 antibody differed depending on the GPC3 expression levels in the cells. In a normal environment where only the peripheral blood cells are present, the positive control group showed a maximum of about 60 % T cell activation, whereas the protein complex of CD3-binding antibody variable region 3-Fc x GPC3 antibody did not significantly activate T cells, with less than 10 % activation. Meanwhile, it was confirmed that, in the presence of the HepG2 cancer cells with high GPC3 expression levels and the Huh7 cancer cells with moderate expression levels, results were similar to the maximum activation of the positive control monoclonal antibody. This suggests that, by weakly regulating the binding affinity of the protein complex of CD3-binding antibody variable region 3-Fc x GPC3 antibody, T cells can be activated only in an environment with the cancer cells and the activation of T cells can be weakened in an environment without the cancer cells. This cancer-specific activation can reduce side effects caused by excessive release of pro-inflammatory cytokines observed with the existing CD3-binding antibodies.

### 2-5. Identification of release level of off-target cytokines by protein complex of CD3-binding antibody variable region 3-Fc x GPC3 antibody

The extent of cytokine release from human peripheral blood mononuclear cells by the protein complex of CD3-binding antibody variable region-Fc x GPC3 antibody was confirmed. Human peripheral blood mononuclear cells (Stemcell Technologies, US) were treated with a CD3 (UCHT1)-binding monoclonal antibody as a positive control group and with a test substance, and cultured. Then, the concentrations of specific cytokines in the collected culture medium were analyzed.

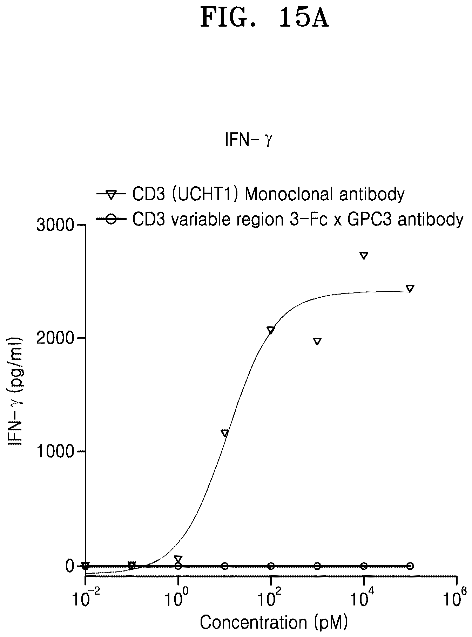

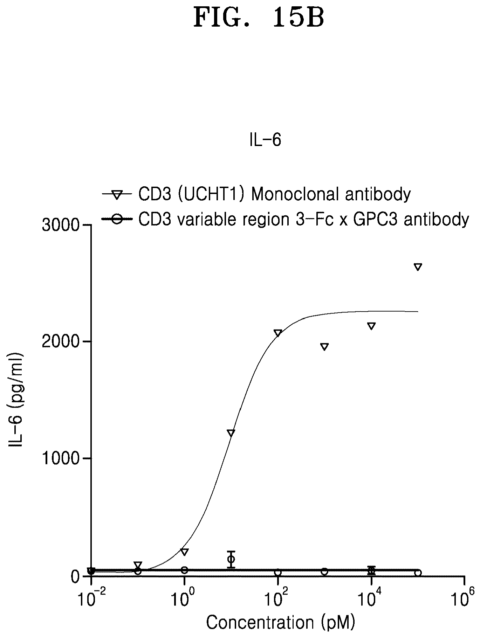

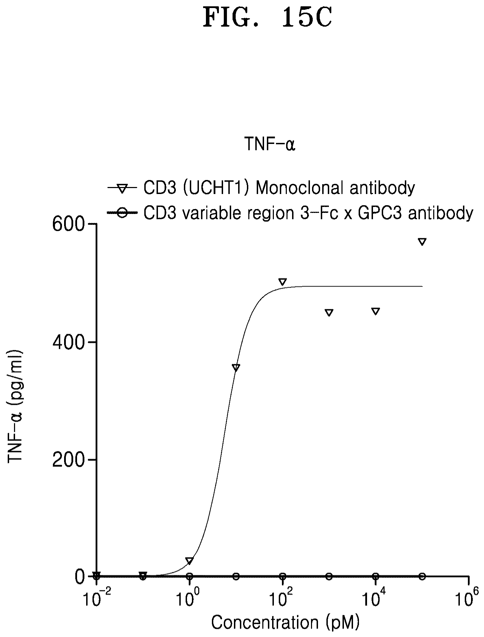

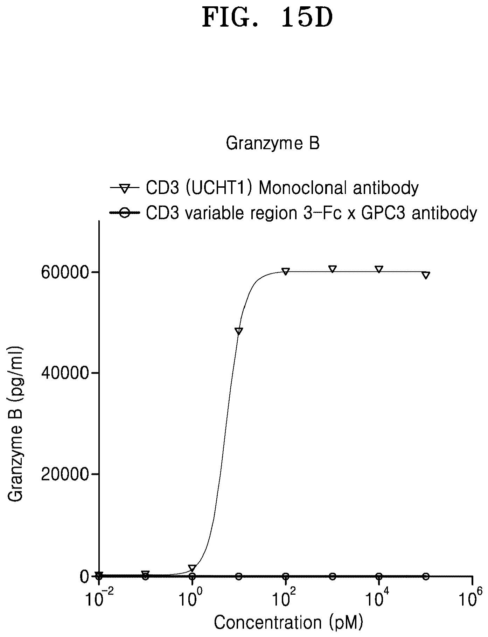

Human peripheral blood mononuclear cells in a frozen state were thawed within 2 minutes in a 37 °C water bath, mixed with RPMI1640 (Thermofisher scientific, US) medium supplemented with 10% FBS (Thermofisher scientific, US), and centrifuged to prepare peripheral blood mononuclear cells. The prepared peripheral blood mononuclear cells were inoculated at a cell count of 2 x 10⁵ cells, treated with a control substance and the protein complex of CD3-binding antibody variable region 3-Fc x GPC3 antibody at various concentrations (serial dilution by 1/10 within a concentration range of 100 nM to 0.01 pM), and then cultured for 48 hours. The results are shown in FIGS. 15A to 15D.

As a result, in the environment without the cancer cells, the positive control group released at least 2,000 pg/mL of IFN-γ, at least 200 pg/mL of IL-6, at least 400 pg/mL of TNF-α, and at least 50,000 pg/mL of Granzyme B. Meanwhile, the protein complex of CD3-binding antibody variable region 3-Fc x GPC3 showed significantly lower release levels of 0.14 to 19.3 pg/mL for these four types of cytokines, representing a significant low release level by 60-fold to about 9,000-fold. This indicates that, due to the protein complex of CD3-binding antibody variable region 3-Fc x GPC3 antibody acting in a target-specific manner, activation of cells other than target cells in the environment without cancer cells is minimized, thereby reducing the release of pro-inflammatory cytokines.

Accordingly, side effects caused by excessive release of pro-inflammatory cytokines observed in the existing CD3-binding antibodies can be reduced, thereby providing a safer therapeutic agent.

### 2-6. Identification of cell-killing effect and level of cytokine release induced by protein complex of CD3-binding antibody variable region-Fc x GPC3 antibody

The cancer cell-killing effect and the extent of IFN-γ release induced by the expression of GPC3 and PD-L1 induced by the protein complex of CD3-binding antibody variable region-3Fc x GPC3 antibody was confirmed. For use as target cells, human liver cancer cell line HepG2 (ATCC, US) expressing high levels of GPC3 was used, and for effector cells, human peripheral blood mononuclear cells (Stemcell Technologies, US) were used, and these cells were co-cultured. Then, the extent of death of cancer cells was analyzed by flow cytometry, and the culture medium was collected to analyze the release level of IFN-γ using a Cytometric Bead Array (CBA) method. For use as a positive control group for the analysis, a CD3-binding single antibody (Biolegend, US) was used, and the activity of the protein complex of CD3-binding antibody variable region 3-Fc x GPC3 antibody in which the binding affinity to the CD3-binding antibody has been regulated was evaluated.

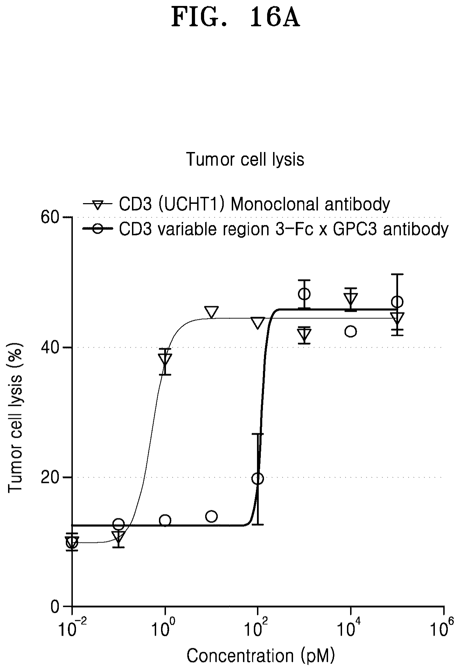

First, to distinguish the HepG2 cells and the human peripheral blood mononuclear cells, the HepG2 cells were treated using a 2 mM cell trace violet-cell proliferation kit (Invitrogen, US), and reacted in an incubator at 37 °C for 10 minutes for fluorescence labeling. The labeled cells were seeded at 1 x 10⁴ cells/well in a 96-well plate and cultured for at least 16 hours to prepare target cells. Human peripheral blood mononuclear cells in a frozen state were thawed within 2 minutes in a 37 °C water bath, mixed with DMEM medium supplemented with 10% FBS, and centrifuged to prepare effector cells. The target cancer cells were treated with the positive control and the protein complex of CD3-binding antibody variable region-3Fc x GPC3 antibody at various concentrations (serial dilution by 1/10 within a concentration range from 100 nM to 0.01 pM). Next, the prepared peripheral blood mononuclear cells were inoculated at 20 times the number of the target cells and co-cultured for 48 hours. To confirm the target cancer cell-killing effect, after co-culture, the target cells were detached using Accutase (BD Bioscience, US), washed with PBS, and then reacted with 7-AAD (Thermofisher, US) at 4 °C for 30 minutes in the dark to label dead cells. Subsequently, flow cytometry was performed using FACSLyric flow cytometry (BD Biosceicne, US). The dead cancer cells were classified as cell trace violet positive and 7-AAD positive, and the results are shown in FIG. 16A.

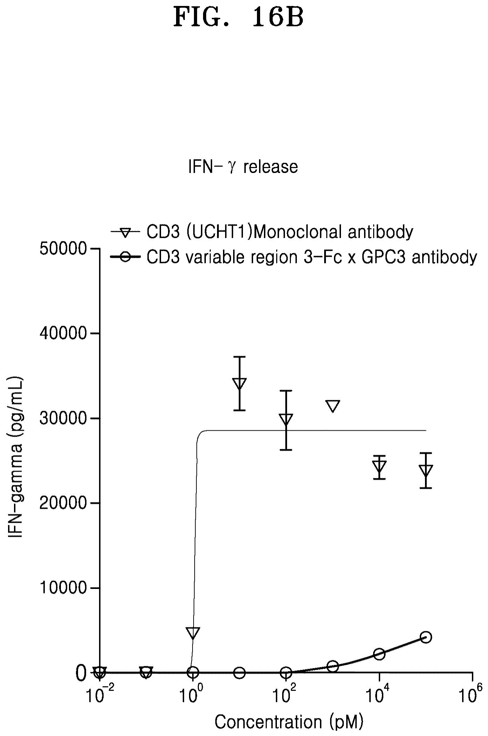

To confirm the extent of cytokine release, after co-culture, the IFN-γ concentration in the collected culture medium was measured. Cytometric bead array (BD Bioscience, US), FACSLyric flow cytometry (BD Bioscience, US), and CBA analysis software (BD Bioscience, US) were used for the measurement, and the results are shown in FIG. 16B.

The test results confirmed that the maximum cytokine release was significantly reduced in the protein complex of CD3-binding antibody variable region 3-Fc x GPC3 antibody compared to the positive control group.

In addition, the cancer cell-killing was similar to that of the positive control group, and the protein complex of CD3-binding antibody variable region 3-Fc x GPC3 antibody showed a similar maximum cancer cell-killing effect in cancer cells. This confirmed that the cancer cell-killing effect in cancer cells was maintained with similar results.

This indicates that the protein complex of CD3-binding antibody variable region 3-Fc x GPC3 antibody in which the binding affinity to CD3 has been regulated reduces the binding strength to CD3, while maintaining tumor lysis effects similar to those of the positive control groups, thereby reducing the maximum level of cytokine release by the death of cancer cells, confirming that a substance with high safety was obtained.

The foregoing descriptions are only for illustrating the disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

1. A CD3 antibody or an antigen-binding fragment thereof, comprising a heavy chain variable region and a light chain variable region represented by the following formulas:
- Formula 1 (heavy chain variable region): 
EVQLVESGGGLVQPGGSLR1LSCAASGX1SFTGYTMNWVRQAP
GKX2LEWVALINPYKGVTTYADSVKGRFTISX3DX4SKNTX5YLQM
NSLRAEDTAVYYCARSGYYGDSDWYFDVWGQGTLVTVSS;
 and
- Formula 2 (light chain variable region): 
DIQMTQSPSSLSASVGDRVTITCRASQDIRNYLNWYQQKPGKA
PKLLIYYTSX6LESGVPSRFSGSGSGTDYTLTISSLQPEDFATYYC
QQGNTLPWTFGX7GTKVEIKRT,
wherein X₁ to X₅ in the heavy chain variable region are each selected from the group consisting of tyrosine (Y), glycine (G), cysteine (C), alanine (A), aspartic acid (D), glutamine (Q), valine (V), lysine (K), arginine(R), phenylalanine (F), asparagine(N), serine (S), and leucine (L), and
wherein X₆ and X₇ in the light chain variable region are each selected from the group consisting of tyrosine (Y), glycine (G), cysteine (C), alanine (A), aspartic acid (D), glutamine (Q), valine (V), lysine (K), arginine (R), phenylalanine (F), asparagine (N), serine (S), and leucine (L).

2. The CD3 antibody or an antigen-binding fragment thereof of claim 1, wherein X₁ to X₅ in the heavy chain variable region are each an amino acid selected from the group consisting of tyrosine (Y), phenylalanine (F), glycine (G), valine (V), arginine (R), lysine (K), asparagine (N), alanine (A), aspartic acid (D), glutamine (Q), leucine (L), and cysteine (C), and
X₆ and X₇ in the light chain variable region are each selected from the group consisting of arginine (R), serine (S), glutamine (Q), and cysteine (C).

3. The CD3 antibody or an antigen-binding fragment thereof of claim 2, wherein, in the heavy chain variable region, X₁ is phenylalanine (F) or tyrosine (Y),
X₂ is glycine (G) or cysteine (C),
X₃ is valine (V), arginine (R), alanine (A), aspartic acid (D), or glutamine (Q),
X₄ is lysine (K) or asparagine (N), and
X₅ is alanine (A) or leucine (L), and
in the light chain variable region, X6 is arginine (R) or serine (S), and
X₇ is glutamine (Q) or cysteine (C).

4. The CD3 antibody or an antigen-binding fragment thereof of claim 3, wherein, in the heavy chain variable region,
X₁ is phenylalanine (F),
X₂ is glycine (G),
X₃ is arginine (R),
X₄ is asparagine (N), and
X₅ is leucine (L), and
in the light chain variable region, X₆ is serine (S), and
X₇ is glutamine (Q).

5. The CD3 antibody or an antigen-binding fragment thereof of claim 3, wherein, in the heavy chain variable region,
X₁ is phenylalanine (F),
X₂ is cysteine (C),
X₃ is arginine (R),
X₄ is asparagine (N), and
X₅ is leucine (L), and
in the light chain variable region, X₆ is serine (S), and
X₇ is cysteine (C).

6. The CD3 antibody or an antigen-binding fragment thereof of claim 3, wherein, in the heavy chain variable region,
X₁ is phenylalanine (F),
X₂ is glycine (G),
X₃ is alanine (A),
X₄ is asparagine (N), and
X₅ is leucine (L), and
in the light chain variable region, X6 is serine (S), and
X₇ is glutamine (Q).

7. The CD3 antibody or an antigen-binding fragment thereof of claim 3, wherein, in the heavy chain variable region,
X₁ is phenylalanine (F),
X₂ is cysteine (C),
X₃ is alanine (A),
X₄ is asparagine (N), and
X₅ is leucine (L), and
in the light chain variable region, X6 is serine (S), and
X₇ is cysteine (C).

8. The CD3 antibody or an antigen-binding fragment thereof of claim 1, wherein the CD3 antibody includes
at least one polypeptide selected from the group consisting of SEQ ID NOs: 1 to 10.

9. The CD3 antibody or an antigen-binding fragment thereof of claim 1, wherein the CD3 antibody has two chains or a single chain.

10. The CD3 antibody or an antigen-binding fragment thereof of claim 9, wherein the CD3 antibody having a single chain has a domain in which the heavy chain variable region and the light chain variable region are linked together by a linker or carrier.

11. The CD3 antibody or an antigen-binding fragment thereof of claim 10, wherein the linker includes a polypeptide represented by SEQ ID NO: 13 or 14.

12. A protein complex comprising a first polypeptide having a first CH3 antibody constant region and a second polypeptide having a second CH3 antibody constant region, wherein the first polypeptide and the second polypeptide form a heterodimer,
at least one selected from the group consisting of an N-terminal and a C-terminal of the first polypeptide comprises an immune checkpoint inhibitor, an antibody or an antigen-binding fragment thereof against a tumor-specific antigen or tumor-associated antigen, a cytokine, a hormone, or an immune cell receptor, and
at least one selected from the group consisting of an N-terminal and a C-terminal of the second polypeptide comprises the CD3 antibody of claim 1, an immune checkpoint inhibitor, an antibody or an antigen-binding fragment thereof against a tumor-specific antigen or tumor-associated antigen, a cytokine, a hormone, or an immune cell receptor.

13. The protein complex of claim 12, wherein the first CH3 antibody constant region includes tryptophan (W) at position 366, and the second CH3 antibody constant region includes serine (S) at position 366, alanine (A) at position 368, and valine (V) at position 407, and
the second CH3 antibody constant region includes glycine (G) or phenylalanine (F) at position 351
(wherein the positions of the amino acids are based on the Kabat EU index).

14. The protein complex of claim 12, wherein the first CH3 antibody constant region includes serine (S) at position 366, alanine (A) at position 368, and valine (V) at position 407, and the second CH3 antibody constant region includes W at position 366; and
the first CH3 antibody constant region includes glycine (G) or phenylalanine (F) at position 351
(wherein the positions of the amino acids are based on the Kabat EU index).

15. The protein complex of claim 12, wherein the first CH3 antibody constant region includes tryptophan (W) at position 366, and the second CH3 antibody constant region includes glycine (G) at position 351, serine (S) at position 366, alanine (A) at position 368, and valine (V) at position 407, and
the first CH3 antibody constant region includes phenylalanine (F) or tryptophan (W) at position 351
(wherein the positions of the amino acids are based on the Kabat EU index).

16. The protein complex of claim 12, wherein the first CH3 antibody constant region includes glycine (G) at position 351, serine (S) at position 366, alanine (A) at position 368, and valine (V) at position 407, and the second CH3 antibody constant region includes tryptophan (W) at position 366, and
the second CH3 antibody constant region includes phenylalanine (F) or tryptophan (W) at position 351
(wherein the positions of the amino acids are based on the Kabat EU index).

17. The protein complex of claim 12, wherein the first CH antibody constant region and the second CH antibody constant region include alanine (A) at positions 234 and 235
(wherein the positions of the amino acids are based on the Kabat EU index).

18. The protein complex of claim 12, wherein the immune checkpoint inhibitors are at least one selected from the group consisting of PD-L1, PD-1, LAG-3, VISTA, BTLA, TIM-3, TIGIT, OX40 and CTLA-4, and KLRG-1.

19. The protein complex of claim 12, wherein the tumor-specific antigen or tumor-associated antigen is at least one selected from the group consisting of EpCAM, mesothelin (MSLN), TRP1, MUC1, PSA, EGFR, HER-2, B7H3, GPC3, CEA, VEGFR, TROP, Claudin18.2, CD20, CD19, CD22, and PSMA.

20. The protein complex of claim 12, wherein the cytokine is at least one selected from the group consisting of IL2, II15, IL17, IL21, and IL12, or a derivative thereof.

21. The protein complex of claim 12, wherein the immune cell receptors are at least one selected from the group consisting of transforming growth factor beta (TGF-β), vascular endothelial growth factor (VEGF), Delta Like Canonical Notch Ligand 4 (DLL-4), CD47, CD28, 41BB, and CD27.

22. A multispecific antibody comprising the CD3 antibody or an antigen-binding fragment thereof of claim 1.

23. A polynucleotide encoding the protein or protein complex of any one of claims 1 to 21.

24. A method of preparing a protein or protein complex, the method comprising transfecting a cell with an expression vector that encodes the protein or protein complex of any one of claims 1 to 21.

25. A pharmaceutical composition for preventing or treating cancer, the pharmaceutical composition comprising the multispecific antibody of claim 22.
